# EUROPEAN PATENT APPLICATION

(11) **EP 4 671 265 A1**
(43) Date of publication of application: **31.12.2025**
(21) Application number: 24185492.6
(22) Date of filing: 28.06.2024
(51) Int. Cl.: C07K 14/005, C12N 15/85

(54) **GENETIC CONSTRUCTS FOR IMPROVED AAV TITERS AND POTENCY**

(71) Applicant: Sartorius Stedim Cellca GmbH, 89081 Ulm (DE)
(72) Inventor: Jung, Ulrike, 89081 Ulm (DE); Ali, Sadfer, 89081 Ulm (DE); Mathias, Sven, 89081 Ulm (DE); Krämer, Jan Niklas, 33758 Schloss Holte-Stukenbrock (DE); Boi, Stefano, 89081 Ulm (DE)
(74) Representative: Cohausz & Florack

(57) **Abstract**

The present invention relates to a rep plasmid comprising at least one long adeno-associated virus replication protein coding sequence encoding at least one long functional rep protein, at least one short adeno-associated virus replication protein coding sequence encoding at least one short functional rep protein, and at least one heterologous element, wherein following transcription the at least one heterologous element results in a ratio of long adeno-associated virus rep mRNA to short adeno-associated virus rep mRNA of about 1:5 to about 1:5000.

## Description

### FIELD OF THE INVENTION

The present invention relates to a rep plasmid comprising at least one long adeno-associated virus replication protein coding sequence encoding at least one long functional rep protein, at least one short adeno-associated virus replication protein coding sequence encoding at least one short functional rep protein, and at least one heterologous element, wherein following transcription the at least one heterologous element results in a ratio of long adeno-associated virus rep mRNA to short adeno-associated virus rep mRNA of about 1:5 to about 1:5000. The invention further relates to a plasmid system for producing an adeno-associated virus particle comprising the rep plasmid of the invention and a transgene plasmid. In another aspect of the invention, a stable or transient cell expression system comprising the plasmid system of the invention is provided. In still another aspect of the invention, a cell comprising the rep plasmid, or the plasmid system of the invention is provided. In still another aspect, the invention provides a kit comprising a stable or transient cell expression system or a cell of the invention. In still another aspect of the invention, a method for producing recombinant adeno-associated viral vectors comprising transfecting cells with the rep plasmid or the plasmid system of the invention is provided.

### BACKGROUND OF THE INVENTION

Several gene delivery techniques have been developed to express a gene of interest in cells, tissues, or organisms, including mammalian and in particular human cells, tissues, or organisms.

Some of these delivery techniques use a viral vector derived from lentivirus, oncoretrovirus, adenovirus, adeno-associated virus or similar viruses. Among these vectors, the adeno-associated virus (AAV) vector has been recognized as a promising tool. Adeno-associated viruses (AAVs) are linear, single-stranded DNA viruses that belong to the parvovirus family. AAVs are infectious to cells of a wide range of species, including humans, and can infect non-dividing cells in which differentiation has ceased, such as blood cells, muscle or nerve cells. In addition, wild-type AAVs are non-pathogenic to humans, and AAV particles are physiochemically very stable. These characteristics have led to the development of recombinant AAVs (rAAVs) as vectors for gene delivery and in particular gene therapy.

The current AAV-based gene therapy market has two FDA-approved AAV-based gene therapies: 1) Luxturna (voretigene neparvovec), approved in 2017 for Leber congenital amaurosis treatment, with subsequent European Commission approval in November 2018, and 2) Zolgensma, which was approved in 2019 for spinal muscular atrophy. The approval of these medications has dramatically impacted the field of AAV-based gene therapies. In addition, AAV has also been studied as a gene therapy tool for cancer treatment (Asaad et al., "AAV genome modification for efficient AAV production").

The single-stranded genome of wild-type AAV comprises rep (replication) and cap (capsid) genes. These genes give rise to several rep and cap proteins through alternative translation start sites and differential splicing. The coding sequences are flanked by inverted terminal repeats (ITRs), leading the AAV genome to form a T-shaped hairpin structure through the ITR at both ends, wherein the linear single-stranded genome between the hairpin structures encodes the rep and cap proteins. The rep gene encodes four proteins (rep78, rep68, rep52, and rep40) for viral genome replication and packaging (replication proteins or rep proteins), while cap expression gives rise to three capsid proteins (cap proteins; VP1, VP2, and VP3), which form the outer capsid shell that protects the AAV genome, as well as being involved in cell binding and internalization.

A recombinant AAV (rAAV), which lacks the wild-type genome, is a protein-based nanoparticle designed to cross the cell membrane where it can transport and deliver its recombinant DNA cargo into the nucleus of a cell. In the absence of the rep proteins, ITR-flanked transgenes encoded by rAAV can form circular concatemers that persist as episomes in the nucleus of transduced cells. Because recombinant episomal DNA does not integrate into the host genome, it will eventually become diluted over time as the cell undergoes repeated rounds of replication. This will eventually lead to loss of the transgene and transgene expression, with the rate of transgene loss depending on the turnover rate of the transduced cell. These characteristics make rAAV ideal for gene therapy applications and AAV-vector-mediated gene delivery was recently approved for the treatment of inherited blindness and spinal muscular atrophy, and long-term therapeutic effects have been achieved for other rare diseases, including hemophilia and Duchenne muscular dystrophy.

Typical recombinant adeno-associated viral vectors (rAAV vectors) have a genome structure in which the rep and cap genes between the ITRs of the wildtype AAV genome are replaced by one or more transgenes to form a transgene plasmid for gene delivery in gene therapy applications. An example of the method of producing an rAAV vector is a method comprising introducing into a host cell a transgene plasmid in which a transgene is inserted between ITRs and introducing a rep plasmid for supplying the rep protein for replication to produce an rAAV vector in the host cell. The rep plasmid can further comprise the cap gene or the cap gene can be located on a separate cap plasmid introduced into the host cell. Furthermore, accessory helper genes from other viruses like Herpes or Adenoviruses can be introduced in the cell to regulate cell metabolism as well as AAV gene expression, prevent apoptosis, and other functions.

While wildtype AAV is capable of providing 100% full and infectious particles, with rAAV the range is usually with 1-30% full (the latter usually achieved rarely and only with very intense effort for particular transgenes and other elements combinations) and only 1 out of 200-1000 full particles infectious. Consequently, when using an rAAV vector for gene therapy applications, it is challenging to produce a sufficiently high titer required for efficient and effective treatment.

The increasing number of clinical and preclinical studies using AAV-based gene therapy necessitates using cost-effective but efficient methods for AAV production. Many studies have been conducted to optimize the AAV production in both upstream and downstream stages, and introducing specific modifications to the AAV genome might help improve production to achieve low production cost and/or efficient and effective treatment.

Rep proteins enable viral genome replication and packaging, however, they are suggested to negatively interfere with rAAV production due to their cytotoxic effects on the producer cells. Particularly, the long rep proteins, i.e., rep78 and rep 68 (or variants thereof)), are reported to exert cytotoxic effects, e.g., by caspase-3 activation resulting in cell death by apoptosis (Schmidt, et al., "Adeno-Associated Virus Type 2 Rep78 Induces Apoptosis through Caspase Activation Independently of p53"; J Virol. 2000 Oct; 74(20): 9441-9450; doi: 10.1128/jvi.74.20.9441-9450.2000). However, the precise roles and effects of the four individual rep proteins in rAAV production are not understood.

Besides low rAAV titers, high levels of product-related impurities, e.g., empty/partially filled viral capsids, during production further challenge rAAV production. Product-related impurities can include AAV empty capsids, encapsidated host cell nucleic acid/helper DNA, and noninfectious AAV capsids. Among these impurities, empty capsids are reported to be the most detrimental to productivity and cause the lot-to-lot variability in products. A purification process that cannot substantially remove this product-related impurity can result in exacerbated immune responses, reduced transduction efficiency and potency. The problem is further exacerbated as during current common purification methods by chromatography the product loss is increasing exponentially with the percentage of empty capsid impurity in the raw bulk product. Accordingly, improving the concentration of viral genomes (i.e., the genomic titer) as well as the full particle (i.e., full capsid) ratio in the raw bulk product is particularly imperative for cost effective gene therapy with AAV and achieving highly pure drug substances for gene therapy applications.

There is therefore an unmet need for improved rAAV production methods and systems that achieve high titers of functional rAAV particles, i.e., rAAV particles with a high potency.

### SUMMARY OF THE INVENTION

Against the aforementioned background, it is therefore an object of the present invention to provide improved means for efficient rAAV production. It is also an object of the present invention to provide rAAV capsids (rAAV particles) which are properly filled with their intended DNA cargo (referred to as full capsids), while the fraction of empty capsids, meaning of capsids that are not filled with their intended DNA cargo, is reduced. A further object of the invention is to provide improved rAAV vectors capable of successfully transducing cells. It is also an object of the invention to provide rAAV vectors with improved transduction efficiency. It is also an object of the invention to provide rAAV vectors with improved potency. It is a further object of the invention to provide rAAV vector production systems with reduced toxicity for the producer cells. It is thus an object of the invention to provide improved plasmid systems for rAAV production that enable production of high titers of rAAV particles with high potency.

These objects are achieved by the invention set forth in the claims and embodiments explained in more detail below.

The invention concerns a rep plasmid comprising:
(i) at least one long adeno-associated virus replication protein coding sequence encoding at least one long functional rep protein;
(ii) at least one short adeno-associated virus replication protein coding sequence encoding at least one short functional rep protein; and
(iii) at least one heterologous element, wherein following transcription the at least one heterologous element results in a ratio of long adeno-associated virus rep mRNA to short adeno-associated virus rep mRNA of about 1:5 to about 1:5000.

The invention further relates to a rep-cap plasmid comprising:
(i) at least one long adeno-associated virus replication protein coding sequence encoding at least one long functional rep protein;
(ii) at least one short adeno-associated virus replication protein coding sequence encoding at least one short functional rep protein;
(iii) at least one adeno-associated virus capsid protein coding sequence encoding at least one functional cap protein; and
(iv) at least one heterologous element, wherein following transcription the at least one heterologous element results in a ratio of long adeno-associated virus rep mRNA to short adeno-associated virus rep mRNA of about 1:5 to about 1:5000.

Furthermore, the invention provides a plasmid system for producing an adeno-associated virus particle comprising:
(i) the rep plasmid of the invention; and
(ii) a transgene plasmid.

The invention achieves efficient rAAV vector production by the rep plasmid of the invention, the rep-cap plasmid of the invention, and/or the plasmid system of the invention. In particular, the plasmids and the plasmid system of the invention enable optimization of the ratio of long rep (rep78, rep68, or variants thereof) to short rep (rep40, rep52, or variants thereof) proteins. The research underlying this invention has surprisingly found that low concentrations of long rep proteins relative to the concentration of short rep proteins results in improved rAAV vector production. In particular, it has been found that a long rep protein to short rep protein mRNA ratio of 1:5 to 1:5000 is beneficial for improving the transducing titer, i.e., the potency, and the viral genome and viral capsid titers. Cells transfected with the rep plasmid of the invention for the production of rAAV particles provide an increased concentration of viral genomes and a higher percentage of full capsids. Furthermore, rAAV particles produced by the rep plasmid of the invention provide an increased potency. The rep plasmid of the invention thus is particularly suitable for producing rAAVs for gene therapy applications, wherein a high titer of functional rAAV vectors is required for efficient and/or effective treatment.

The invention also concerns a stable or transient cell expression system comprising the plasmid system of the invention and a cell, preferably a cell line. The invention further provides a cell comprising the rep plasmid of the invention, the plasmid system of the invention, or the rep-cap plasmid of the invention.

The invention further provides a kit comprising a stable or transient cell expression system of the invention or a cell of the invention, and a cell culture medium. This kit provides improved means for efficient rAAV vector production through the rep plasmid of the invention, the plasmid system of the invention, or the rep-cap plasmid of the invention.

The invention further provides a method of producing recombinant adeno-associated viral vectors comprising:
(i) Transfecting cells with the rep plasmid of the invention, the plasmid system of the invention, or the rep-cap plasmid of the invention;
(ii) culturing the transfected cells to produce said adeno-associated viral vectors; and
(iii) isolating said recombinant adeno-associated viral vectors.

Advantageous embodiments of the invention are indicated in the dependent claims and in the following.

### DETAILED DESCRIPTION

Although certain embodiments of the present invention are described in detail below, it is to be understood that this invention is not limited to the particular embodiments, methodologies, protocols and reagents described herein as these may vary within the scope set by the claims. It is also to be understood that terminology used herein is for the purpose of describing particular embodiments only, and is not intended to limit the scope of the present invention which is defined by the appended claims. Unless defined otherwise, all technical and scientific terms used herein have the same meanings as commonly understood by one of ordinary skill in the art.

In the following description, certain elements of the present invention will be described. These elements may be discussed with specific embodiments, however, it should be understood that they may be combined in any manner and in any number to create additional embodiments. The variously described examples, features and particular embodiments should not be construed to limit the present invention to only the explicitly described embodiments or to the explicitly described combination of features. This description should be understood to disclose and encompass embodiments which combine the explicitly described embodiments with any number of the disclosed and/or preferred elements. Furthermore, any permutations and combinations of all described elements in this application should be considered disclosed by this description unless the context indicates otherwise.

The above objects are achieved by the following embodiments in accordance with the invention:
1. A rep plasmid comprising:
   (i) at least one long adeno-associated virus replication protein coding sequence encoding at least one long functional rep protein;
   (ii) at least one short adeno-associated virus replication protein coding sequence encoding at least one short functional rep protein; and
   (iii) at least one heterologous element, wherein following transcription the at least one heterologous element results in a ratio of long adeno-associated virus rep mRNA to short adeno-associated virus rep mRNA of about 1:5 to about 1:5000.
2. The rep plasmid according to embodiment 1, wherein the ratio of long adeno-associated virus replication mRNA to short adeno-associated virus replication mRNA is
   (i) about 1:5 to about 1:300, preferably about 1:5 to about 1:250, more preferably about 1:5 to about 1:200, even more preferably about 1:5 to about 1:150, and most preferably about 1:5 to about 1:100, optionally, wherein the long adeno-associated virus rep protein mRNA level is high; and/or
   (ii) about 1:5 to about 1:5000, preferably about 1:20 to about 1:2500, more preferably about 1:50 to about 1:2000, and most preferably about 1: 100 to about 1: 1500, optionally, wherein the long adeno-associated virus rep protein mRNA level is low.
3. The rep plasmid according to any one of embodiments 1 or 2, wherein after transcription of the rep protein coding sequence, the ratio of long adeno-associated virus replication protein mRNA to short adeno-associated virus replication protein mRNA is
   (i) about 1:5 to about 1:300, preferably about 1:5 to about 1:250, more preferably about 1:5 to about 1:200, even more preferably about 1:5 to about 1:150, and most preferably about 1:5 to about 1:100, optionally, wherein the long adeno-associated virus rep protein mRNA level is high; and/or
   (ii) about 1:5 to about 1:5000, preferably about 1:20 to about 1:2500, more preferably about 1:50 to about 1:2000, and most preferably about 1: 100 to about 1: 1500, optionally, wherein the long adeno-associated virus rep protein mRNA level is low.
4. The rep plasmid according to any one of embodiments 1-3, wherein after translation, the ratio of long adeno-associated virus replication proteins to short adeno-associated virus replication proteins is
   (i) about 1:5 to about 1:300, preferably about 1:5 to about 1:250, more preferably about 1:5 to about 1:200, even more preferably about 1:5 to about 1:150, and most preferably about 1:5 to about 1:100, optionally, wherein the long adeno-associated virus rep protein level is high; and/or
   (ii) about 1:5 to about 1:5000, preferably about 1:20 to about 1:2500, more preferably about 1:50 to about 1:2000, and most preferably about 1: 100 to about 1: 1500, optionally, wherein the long adeno-associated virus rep protein level is low.
5. The rep plasmid according to any one of embodiments 1-4, wherein the at least one heterologous element is a heterologous promoter, preferably operatively linked to the long adeno-associated virus replication protein coding sequence.
6. The rep plasmid according to embodiment 5, wherein the heterologous promoter is characterized by a decreased promoter strength as compared to the native promoter of the short adeno-associated virus replication protein coding sequence.
7. The rep plasmid according to any one of embodiments 5 or 6, wherein the heterologous promoter is selected from a MMTV promoter, a CGA promoter, a DNAH17 promoter, and an AMH promoter.
8. The rep plasmid according to any one of embodiments 1-7, wherein the short adeno-associated virus replication protein coding sequence is operatively linked to a promoter, preferably a native or heterologous promoter.
9. The rep plasmid according to embodiment 8, wherein the promoter is selected from a p19 promoter, a EF1A promoter, a TMBIM6 promoter, and a GAPDH promoter.
10. The rep plasmid according to any one of embodiments 5-9, wherein the promoters are constitutive, inducible, or repressible promoters.
11. The rep plasmid according to embodiments 1-10, wherein the long adeno-associated virus replication protein coding sequence encodes at least one long functional rep protein selected from rep68 and rep78 or artificial variants thereof, preferably selected from rep68 or an artificial variant thereof.
12. The rep plasmid according to any one of embodiments 1-11, wherein the short adeno-associated virus replication protein coding sequence encodes at least one short functional rep protein selected from rep40 and rep52 or artificial variants thereof, preferably selected from rep52 or an artificial variant thereof.
13. The rep plasmid according to any one of embodiments 1-12, wherein the long and/or short adeno-associated virus replication protein coding sequences are independently derived from an AAV serotype selected from the group consisting of AAV-1, AAV-2, AAV-3, AAV-4, AAV-5, AAV-6, AAV-7, and AAV-8, preferably selected from AAV2.
14. The rep plasmid according to any one of embodiments 11-13, wherein the rep78 protein comprises or consists of the SEQ ID NO: 1 or a sequence having at least 60%, at least 70%, at least 80%, or at least 90% identity to said SEQ ID NO.
15. The rep plasmid according to any one of embodiments 11-14, wherein the rep68 protein comprises or consists of the SEQ ID NO: 2 or a sequence having at least 60%, at least 70%, at least 80%, or at least 90% identity to said SEQ ID NO.
16. The rep plasmid according to any one of embodiments 12-15, wherein the rep52 protein comprises or consists of the SEQ ID NO: 3 or a sequence having at least 60%, at least 70%, at least 80%, or at least 90% identity to said SEQ ID NO.
17. The rep plasmid according to any one of embodiments 12-16, wherein the rep40 protein comprises or consists of the SEQ ID NO: 4 or a sequence having at least 60%, at least 70%, at least 80%, or at least 90% identity to said SEQ ID NO.
18. The rep plasmid according to any one of embodiments 1-17, wherein the rep plasmid comprises a 5'UTR upstream of the long adeno-associated virus replication protein coding sequence, preferably derived from the sequence located between the native p5 promoter and the coding sequence of the wildtype long adeno-associated virus replication protein.
19. The rep plasmid according to any one of embodiments 1-18, wherein the rep plasmid comprises a 5'UTR upstream of the short adeno-associated virus replication protein coding sequence, preferably derived from the sequence located between the native p 19 promoter and the coding sequence of the wildtype short adeno-associated virus replication protein.
20. The rep plasmid according to any one of embodiments 1-19, wherein the rep plasmid comprises a 3'UTR downstream of the long and/or short adeno-associated virus replication protein coding sequence, preferably derived from the sequence located between the wildtype capsid protein coding sequence and the wildtype 3' ITR.
21. The rep plasmid according to any one of embodiments 1-20, wherein the rep plasmid comprises one or more polyadenylation signal sequences.
22. The rep plasmid according to any one of embodiments 1-21, wherein the at least one heterologous element is a heterologous polyadenylation signal sequence.
23. The rep plasmid according to any one of embodiments 1-22, wherein the rep plasmid comprises at least one adeno-associated virus capsid protein coding sequence encoding at least one functional cap protein.
24. The rep plasmid according to embodiment 23, wherein the adeno-associated virus capsid protein coding sequence encodes at least one functional cap protein selected from VP1, VP2, and VP3, or artificial variants thereof.
25. The rep plasmid according to any one of embodiments 23 or 24, wherein the adeno-associated virus capsid protein coding sequence is derived from an AAV serotype selected from the group consisting of AAV-1, AAV-2, AAV-3, AAV-4, AAV-5, AAV-6, AAV-7, and AAV-8, preferably selected from AAV2.
26. The rep plasmid according to any one of embodiments 24 or 25, wherein the VP1 protein comprises or consists of the SEQ ID NO: 5 or a sequence having at least 60%, at least 70%, at least 80%, or at least 90% identity to said SEQ ID NO.
27. The rep plasmid according to any one of embodiments 24-26, wherein the VP2 protein comprises or consists of the SEQ ID NO: 6 or a sequence having at least 60%, at least 70%, at least 80%, or at least 90% identity to said SEQ ID NO.
28. The rep plasmid according to any one of embodiments 24-27, wherein the VP3 protein comprises or consists of the SEQ ID NO: 7 or a sequence having at least 60%, at least 70%, at least 80%, or at least 90% identity to said SEQ ID NO.
29. The rep plasmid according to any one of embodiments 1-28, wherein the rep plasmid comprises at least one accessory protein coding sequence encoding at least one functional accessory protein.
30. The rep plasmid according to embodiment 29, wherein the accessory protein coding sequence encodes at least one functional accessory protein selected from AAP, MAAP, and X, or artificial variants thereof.
31. A plasmid system for producing an adeno-associated virus particle comprising:
   (i) the rep plasmid of any one of embodiments 1-30; and
   (ii) a transgene plasmid.
32. The plasmid system according to embodiment 31, wherein the plasmid system further comprises a helper plasmid.
33. The plasmid system according to embodiment 32, wherein the helper plasmid comprises one or more coding sequences encoding for E1a, E1b, E2A, L4 22k, L4 33k, E4orf3, E4orf6, E4orf6-7 fusion, or virus-associated RNA or any combination thereof.
34. The plasmid system according to embodiment 33, wherein the helper plasmid preferably comprises one or more coding sequences encoding for E2A, E4orf6, E4orf6-7 fusion, or virus-associated RNAs or any combination thereof.
35. The plasmid system according to any one of embodiments 33 or 34, wherein the helper plasmid comprises one or more virus-associated RNAs.
36. The plasmid system according to any one of embodiments 31-35, wherein the transgene plasmid comprises a promoter, a transgene, a polyadenylation signal sequence, and 5' and 3' inverted terminal repeats,
   wherein the transgene plasmid is selected from one of the following:
   (i) conventional single-stranded genome recombinant adeno-associated virus, or
   (ii) self-complementary genome recombinant adeno-associated adenovirus.
37. The plasmid system according to embodiment 36, wherein the promoter is a CMVie promoter.
38. The plasmid system according to any one of embodiments 36 or 37, wherein the transgene is a reporter gene, preferably the reporter gene can be detected by antibody-based assays, more preferably the reporter gene is a fluorescent molecule, a beta-galactosidase, luciferase, or glutathione S-transferase.
39. The plasmid system according to any one of embodiments 36-38, wherein the polyadenylation signal sequence a SV40 poly(A) signal sequence.
40. The plasmid system according to any one of embodiments 36-39, wherein the 3' and 5' inverted terminal repeats are independently derived from an ITR sequence of an AAV serotype selected from the group consisting of AAV-1, AAV-2, AAV-3, AAV-4, AAV-5, AAV-6, AAV-7, and AAV-8, preferably selected from AAV2.
41. The plasmid system according to any one of embodiments 36-40, wherein at least one inverted terminal repeat, preferably the 5' inverted terminal repeat, has a deletion compared to the native inverted terminal repeats.
42. The plasmid system according to any one of embodiments 31-41, wherein the plasmid system further comprises a cap plasmid.
43. The plasmid system according to embodiment 42, wherein the cap plasmid comprises at least one adeno-associated virus capsid protein coding sequence encoding at least one functional cap protein.
44. The plasmid system according to any one of embodiments 31-43, wherein the rep plasmid comprises at least one adeno-associated virus capsid protein coding sequence encoding at least one functional cap protein.
45. The plasmid system according to any one of embodiments 43 or 43, wherein the adeno-associated virus capsid protein coding sequence encodes at least one functional cap protein selected from VP1, VP2, and VP3, or artificial variants thereof.
46. The plasmid system according to any one of embodiments 43-45, wherein the adeno-associated virus capsid protein coding sequence is derived from an AAV serotype selected from the group consisting of AAV-1, AAV-2, AAV-3, AAV-4, AAV-5, AAV-6, AAV-7 and AAV-8, preferably selected from AAV2.
47. The plasmid system according to any one of embodiments 42-46, wherein the cap plasmid comprises at least one accessory protein coding sequence encoding at least one functional accessory protein.
48. The plasmid system according to any one of embodiments 31-47, wherein the rep plasmid comprises at least one accessory protein coding sequence encoding at least one functional accessory protein.
49. The plasmid system according to any one of embodiments 47 or 48, wherein the accessory protein coding sequence encodes at least one functional accessory protein selected from AAP, MAAP, and X, or artificial variants thereof.
50. A rep-cap plasmid comprising:
   (i) at least one long adeno-associated virus replication protein coding sequence encoding at least one long functional rep protein;
   (ii) at least one short adeno-associated virus replication protein coding sequence encoding at least one short functional rep protein;
   (iii) at least one adeno-associated virus capsid protein coding sequence encoding at least one functional cap protein; and
   (iv) at least one heterologous element, wherein following transcription the at least one heterologous element results in a ratio of long adeno-associated virus rep mRNA to short adeno-associated virus rep mRNA of about 1:5 to about 1:5000.
51. A stable or transient cell expression system comprising the plasmid system of any one of embodiments 31-49 and a cell line.
52. A cell comprising the rep plasmid of any one of embodiments 1-30, the plasmid system of any one of embodiments 31-49, or the rep-cap plasmid of embodiment 50.
53. A kit comprising the stable or transient cell expression system of embodiment 51, or a cell of embodiment 52, and a cell culture medium.
54. The kit according to embodiment 53, wherein the kit further comprises media feeds, media additives or transfection reagents or any combination thereof.
55. The kit according to any one of embodiments 53 or 54, wherein the kit further comprises a manual.
56. A method of producing recombinant adeno-associated viral vectors comprising:
   (i) Transfecting cells with the rep plasmid of any one of embodiments 1-30, the plasmid system of any one of embodiments 31-49, or the rep-cap plasmid of any one of embodiments 50;
   (ii) culturing the transfected cells to produce said adeno-associated viral vectors; and
   (iii) isolating said recombinant adeno-associated viral vectors.
57. The method according to embodiment 56, wherein the cells are HEK293 cells, HELA cells, CHO cells, insect cells, or derivatives thereof.

### Definitions

The terms indicated for explanation of the invention have the following meaning, unless otherwise indicated in the description or the claims. Additional definitions are set forth throughout the detailed description.

The "3'UTR sequence" is a 3' untranslated region known to regulate mRNA-based processes, such as mRNA localization, mRNA stability, and translation. In addition, 3' UTRs can establish 3' UTR-mediated protein-protein interactions (PPIs), and thus can transmit genetic information encoded in 3' UTRs to proteins. This function has been shown to regulate diverse protein features, including protein complex formation or posttranslational modifications, but is also expected to alter protein conformations.

The "5' UTR sequence" is a 5'-untranslated region which lies within the noncoding genome upstream of a coding sequence and plays an important role in regulating gene expression. Within 5'-UTR sequences may be numerous cis-regulatory elements present that can interact with the transcriptional machinery to regulate mRNA abundance. The 5'-untranslated region may contain various RNA-based regulatory elements including the secondary structures, RNA-binding protein motifs, upstream open-reading frames (uORFs), internal ribosome entry sites, terminal oligo pyrimidine (TOP) tracts, and G-quadruplexes. These elements can alter the efficiency of mRNA translation; some can also affect mRNA transcript levels via changes in stability or degradation.

Terms "a" and "an" and "the" and similar reference used in the context of describing the invention (especially in the context of the claims) are to be construed to cover both the singular and the plural, unless otherwise indicated herein or clearly contradicted by context.

The terms "about" or "approximately" as used herein denotes a range of ±10% of a reference value. For examples, "about 10" defines a range of 9 to 11. In general, those skilled in the art, familiar with the context, will appreciate the relevant degree of variance encompassed by "about" or "approximately" in that context.

As used herein, the term "adeno-associated virus" (AAV), includes but is not limited to, AAV type 1 (e.g., AAV of serotype 1, also referred to as AAV1), AAV type2 (e.g, AAV2), AAV type 3 (e.g, AAV3, including types 3A and 3B, AAV3A and AAV3B), AAV type 4 (e.g, AAV4), AAV type 5 (e.g, AAV5), AAV type 6 (e.g, AAV 6), AAV type 7 (e.g, AAV7), AAV type 8 (e.g, AAV8), AAV type 9 (e.g, AAV9), AAV type 10 (e.g, AAV10), AAV type 11 (e.g, AAV 11), AAV type 12 (e.g, AAV 12), AAV type 13 (e.g, AAV 13), AAV type rh32.33 (e.g, AAVrh32.33), AAV type rh8 (e.g, AAVrh8), AAV type rhlO (e.g, AAVrhlO), AAV type rh74 (e.g, AAVrh74), AAV type hu.68 (e.g, AAVhu.68), avian AAV (e.g, AAAV), bovine AAV (e.g, BAAV), canine AAV, equine AAV, ovine AAV, snake AAV, bearded dragon AAV, AAV2i8, AAV2g9, AAV-LK03, AAV7m8, AAV Anc80, AAV PHP.B, and any other AAV now known or later discovered.

The term "adenoviral late genes" as used herein refers to adenovirus genes that are expressed at a later stage in the adenoviral reproduction cycle, i.e., after the expression of the early adenoviral genes, i.e., after the expression of the E1A, E1B, E2A, E2B, E3, and E4 genes. Without wishing to be bound by theory, it is contemplated that the adenoviral late genes encode for adenoviral capsid proteins and are involved in the assembly of adenoviral capsids.

Also as used herein, "and/or" refers to and encompasses any and all possible combinations of one or more of the associated listed items, as well as the lack of combinations when interpreted in the alternative ("or"). The use of the alternative (e.g., "or") should be understood to mean either one, both, or any combination thereof of the alternatives.

A "cap plasmid" is a plasmid comprising a capsid protein coding sequence encoding for a functional cap protein. The terms "cap plasmid" and "capsid plasmid" can be used interchangeably herein.

Unless expressly specified otherwise, the term "comprising" is used in the context of the present disclosure to indicate that further members may optionally be present in addition to the members of the list introduced by "comprising". It is, however, contemplated as specific embodiments of the present invention that each time the term "comprising" is used, this shall also encompass the possibility of no further members being present, i.e., for the purpose of such specific embodiments "comprising" can be understood as having the meaning of "consisting of'.

As used herein "derived from" in the context of a nucleic acid or amino acid sequence means that the sequence is identical to a sequence from which it is derived or has a specified percentage of amino acid residues or nucleotides that are the same as the sequence from which it is derived. The specified percentage can be about at least 60%, preferably at least 65%, at least 70%, at least 75%, at least 80%, at least 85%, at least 90%, at least 91%, at least 92%, at least 93%, at least 94%, at least 95%, at least 96%, at least 97%, at least 98%, at least 99% over a specified sequence, when compared and aligned for maximum correspondence over a comparison window or designated region as measured using a BLAST or BLAST 2.0 sequence comparison algorithms with default parameters, or by manual alignment and visual inspection. For example, the 5'UTR of the long adeno-associated virus replication protein coding sequence can be derived from a specific sequence of e.g., AAV2, preferably J01901.1 AAV2, e.g., the 5'UTR sequence can be identical to the 5'UTR sequence present between the native p5 promoter and the e.g., the coding sequence of the long adeno-associated replication protein, comprised in the AAV2 genome, preferably J01901.1 AAV2 genome, or can be at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, or at least 99% identical to the 5'UTR sequence comprised in the AAV2 genome, preferably J01901.1 AAV2 genome. For example, the 5'UTR of the short adeno-associated virus replication protein coding sequence can be derived from a specific sequence of e.g., AAV2, preferably J01901.1 AAV2, e.g., the 5'UTR sequence can be identical to the 5'UTR sequence present between the p19 promoter and the e.g., the coding sequence of the short adeno-associated replication protein, comprised in the AAV2 genome, preferably J01901.1 AAV2 genome, or can be at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, or at least 99% identical to the 5'UTR sequence comprised in the AAV2 genome, preferably J01901.1 AAV2 genome. The 3'UTR of the long and/or short adeno-associated replication coding sequence can be derived from a specific sequence of e.g., AAV2, preferably J01901.1 AAV2, e.g., the 3' UTR sequence can be identical to the 3' UTR sequence present between the capsid protein coding sequence and the 3'ITR comprised in the AAV2 genome, preferably J01901.1 AAV2 genome, or can be at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, or at least 99% identical to the 3'UTR sequence comprised in the AAV2 genome, preferably J01901.1 AAV2 genome. In the context of this disclosure, it is understood that "J01901.1" refers to the GenBank accession code "J01901.1" of the wild-type AAV2 complete genome set forth in SEQ ID NO: 25.

Herein, the term "DNA" relates to a nucleic acid molecule which is entirely or at least substantially composed of deoxyribonucleotide residues. In preferred embodiments, the DNA contains all or a majority of deoxyribonucleotide residues. As used herein, "deoxyribonucleotide" refers to a nucleotide which lacks a hydroxyl group at the 2'-position of a β-D-ribofuranosyl group. DNA encompasses without limitation, double stranded DNA, single stranded DNA, isolated DNA such as partially purified DNA, essentially pure DNA, synthetic DNA, recombinantly produced DNA, as well as modified DNA that differs from naturally occurring DNA by the addition, deletion, substitution and/or alteration of one or more nucleotides. Such alterations may refer to addition of non-nucleotide material to internal DNA nucleotides or to the end(s) of DNA. It is also contemplated herein that nucleotides in DNA may be non-standard nucleotides, such as chemically synthesized nucleotides or ribonucleotides. For the present disclosure, these altered DNAs are considered analogs of naturally-occurring DNA. A molecule contains "a majority of deoxyribonucleotide residues" if the content of deoxyribonucleotide residues in the molecule is more than 50% (such as at least 55%, at least 60%, at least 65%, at least 70%, at least 75%, at least 80%, at least 85%, at least 90%, at least 95%, at least 96%, at least 97%, at least 98%, at least 99%), based on the total number of nucleotide residues in the molecule. The total number of nucleotide residues in a molecule is the sum of all nucleotide residues (irrespective of whether the nucleotide residues are standard (i.e., naturally occurring) nucleotide residues or analogs thereof).

DNA may be recombinant DNA and may be obtained by cloning of a nucleic acid, in particular cDNA. The cDNA may be obtained by reverse transcription of RNA.

A "feed" or "supplement" as used herein refers to a composition when added to cells in standard culture may be beneficial for its maintenance, or expansion, or growth, or viability, or affects its cell performance, or increases culture longevity or maintaining cells in a pseudo-stationary phase wherein product expression continues, or results in an increase in final product titer. A feed or supplement may be used interchangeably in this disclosure and refers to solid and liquid formats (including agglomerated formats) of media components comprising one or more amino acids, sugars, vitamins, buffers, sometimes, peptides, hydrolysates, fractions, growth factors, hormones, etc. required to rebalance or replenish or to modulate the growth or performance of a cell in culture, or a cell culture system. A feed or supplement may be distinguished from a cell culture medium in that it is added to a cell culture medium that can culture a cell. As would be understood by one of skill in the art, sometimes a feed/supplement may comprise mainly those amino acids, sugars, vitamins, buffers, etc. required to rebalance or replenish or modulate the growth or performance of a cell in culture, or a cell culture system. A feed or supplement may or may not be concentrated or may be partially concentrated for certain components only.

As used herein, the term "gene" refers to the segment of a DNA molecule that codes for a polypeptide chain (e.g., the coding region). In some embodiments, a gene is positioned by regions immediately preceding, following, and/or intervening the coding region that are involved in producing the polypeptide chain (e.g., regulatory elements such as a promoter, enhancer, polyadenylation sequence, 5'-untranslated region (5'UTR), 3'-untranslated region (3'UTR), or intron).

As used herein, the term "encode" or "encoding" refers to sequence information of a first molecule that guides production of a second molecule having a defined sequence of nucleotides (e.g., mRNA) or a defined sequence of amino acids. For example, a DNA molecule can encode an RNA molecule (e.g., by a transcription process that includes a DNA-dependent RNA polymerase enzyme). A coding sequence encoding a protein is a sequence that guides production of said protein. Thus, a coding sequence, a gene, a cDNA, or a single-stranded RNA (e.g., an mRNA) encodes a polypeptide if transcription and translation of mRNA corresponding to that gene produces the polypeptide in a cell or other biological system. In some embodiments, a coding sequence encoding a target polypeptide refers to a coding strand, the nucleotide sequence of which can be identical to the mRNA sequence of such a target polypeptide. In some embodiments, a coding sequence encoding a target polypeptide refers to a non-coding strand of such a target polypeptide agent, which may be used as a template for transcription of a gene or cDNA. As is understood in the art, the phrase "coding sequence encoding a peptide or protein" means that the plasmid containing the coding sequence, if present in the appropriate environment, for example within a cell and/or in a cell-free translation system, can direct the assembly of amino acids to produce the peptide or protein via a process of translation.

A functional replication protein in context of the invention is a replication protein involved in AAV replication and/or AAV assembly. The terms "replication protein" and "rep protein" are used synonymously in the context of the present invention. It is within the abilities of the skilled person to determine whether a given rep protein is functional. The functionality of rep78, rep68, rep52, and/or rep40 polypeptides or artificial variants thereof may be determined by the skilled person using PCR-based techniques, e.g., by droplet digital PCR (ddPCR) or real-time PCR (qPCR), or by Western Blot. In some embodiments, the functional rep protein is involved in AAV replication and viral assembly. In some embodiments, the functional rep protein is involved in AAV replication. In some embodiments, the functional rep protein is involved in AAV virion assembly. In some embodiments, the functional rep protein binds to DNA. In some embodiments, the functional rep protein does not bind to DNA.

The term "level" as used herein in conjunction with the long adeno-associated virus rep protein or long adeno-associated virus rep protein mRNA refers to the absolute concentration or content of such protein or mRNA. A high level preferably refers to a comparably high concentration of content of such protein or mRNA, whereas a low level preferably refers to a comparably low concentration of content of such protein or mRNA. Such low concentration may be 50% or less of the high level, preferably 25% or less of the high level, more preferably 10% or less of the high level.

A functional capsid protein in context of the invention is a capsid protein capable of forming a capsid. The terms "capsid protein" and "cap protein" are used synonymously in the context of the present disclosure. A functional cap protein can be any of VP1, VP2, VP3 or artificial variants thereof capable of forming a capsid. It is within the abilities of the skilled person to determine whether a given cap protein is functional. For example, the functionality of a cap protein may be determined in a test two- or three-plasmid system, which comprises a test plasmid comprising the cap gene(s) which encode the cap protein(s) whose functionality is to be determined. Besides the cap gene(s) on the test plasmid, the test two- or three-plasmid system is identical to a reference two- or three-plasmid system. The reference two- or three-plasmid system further comprises all necessary genetic information for the production of rAAV particles. The reference two or three-plasmid system preferably comprises the wild-type cap coding sequence preferably encoding one or more of VP1, VP2, and VP3 having the amino acid sequences shown in SEQ ID NO: 5-7. A cap protein may be considered functional if the rAAV production provided by the test two- or three-plasmid system comprising the cap protein(s) whose activity is to be determined is at least 10%, preferably at least 5%, more preferably at least 1% of the level of rAAV production of the reference two- or three-plasmid system. The skilled person knows how to determine rAAV particle formation and thus formation of functional capsids, e.g., by Enzyme-linked Immunosorbent Assay (ELISA), differential scanning fluorimetry (DSF), chromatographic, or spectroscopic methods (e.g., bio-layer interferometry and/or surface plasmon resonance spectroscopy). For example, methods to verify functional capsid assembly are described in Green and Kelvin, "Analytical methods to characterize recombinant adeno-associated virus vectors and the benefit of standardization and reference materials ", Curr Opin Biotechnol. 2021 Oct; 71: 65-76, doi: 10.1016/j .copbio.2021.06.025.

The term "helper" is not intended to be limiting. Accordingly, a "helper plasmid" is any plasmid that comprises at least one helper virus gene. In some embodiments, a helper virus gene can be selected from E1a, E1b, E2A, L4 22k, L4 33k, E4orf3, E4orf6, E4orf6-7 fusion, or virus-associated RNA or any combination thereof.

The term "heterologous element" in the context of the invention refers to a polynucleotide sequence that can influence the ratio of long rep protein mRNA to short rep protein mRNA and is not present in the native AAV genome, preferably in the native AAV2 genome. In some embodiments, the heterologous element is a non-coding DNA sequence. In some embodiments, the heterologous element is derived from a different organism such as another virus or a mammal e.g., a mouse mammary tumor virus or rabbit. In some embodiments, the heterologous element is selected from a heterologous promoter, a heterologous polyadenylation signal sequence, and a heterologous nuclear export enhancer (e.g., a woodchuck post-transcriptional element). For example, the heterologous element can be a heterologous promoter operatively linked to the long adeno-associated virus rep protein coding sequence. "Operatively linked" in this context means that the long adeno-associated virus rep protein coding sequence is covalently linked to the heterologous promoter in such a way that the expression or transcription of the long rep protein coding sequence is under the influence or the control of the heterologous element, particularly the heterologous promoter. In some embodiments, the rep plasmid of the invention can comprise more than one heterologous element. For example, the rep-plasmid can comprise a heterologous promoter and a heterologous polyadenylation signal sequence that can preferably both influence the ratio of long rep protein mRNA to short rep protein mRNA . In some embodiments, the heterologous element refers to an artificial variant of a polynucleotide sequence present in the native AAV genome, preferably in the native AAV2 genome. In some embodiments, the heterologous promoter is selected from an artificial variant of a native promoter present in the AAV genome, preferably in the AAV2 genome, e.g., an artificial variant of the p5 promoter.

The terms "identical" or percent (%) "identity," in the context of two or more nucleic acids or peptide sequences, refer to two or more sequences or subsequences that are the same or have a specified percentage of amino acid residues or nucleotides that are the same (i.e., about 60% identity, preferably 65%, 70%, 75%, 80%, 85%, 90%, 91%, 92%, 93%, 94%, 95%, 96%, 97%, 98%, 99%, or higher identity over a specified region, when compared and aligned for maximum correspondence over a comparison window or designated region) as measured using a BLAST or BLAST 2.0 sequence comparison algorithms with default parameters, or by manual alignment and visual inspection.

Inverted terminal repeats (ITRs) are guanine-cytosine-rich structures involved in the replication and encapsidation of the AAV genome, along with its integration in and excision from the host genome. ITRs are natural AAV-derived DNA sequences conserved in recombinant AAV (rAAV), as they allow its replication, encapsidation, and long-term maintenance and expression in target cells. ITRs can be incomplete, truncated, and/or modified. An ITR can be a truncated, meaning one or more nucleotides of the natural sequence can be deleted. An ITR can be modified, meaning an ITR can comprise one or more nucleotides of non-ITR sequences. The non-ITR sequence can be inserted within the ITR sequence or the non-ITR sequence can be added at one or both ends of the ITR sequence. The modified ITR sequence can comprise substitution wherein one or more nucleotides are exchanged. It has been shown that rAAV genomes can be replicated, even with incomplete, truncated, or modified ITR sequences. It is understood that the present invention is not limited to a specific ITR sequence and any ITR sequence now known or later discovered can be used.

As used herein, a nucleic acid sequence (e.g., a coding sequence) and regulatory sequences (e.g., the heterologous element disclosed herein) are said to be "operatively linked" when they are covalently linked in such a way as to place the expression or transcription of the nucleic acid sequence under the influence or control of the regulatory sequences. If it is desired that the nucleic acid sequence be translated into a functional protein, two DNA sequences are said to be operatively linked if induction of a promoter in the 5' regulatory DNA sequences results in the transcription of the coding DNA sequence and if the nature of the linkage between the two DNA sequences does not (1) result in the introduction of a frame-shift mutation, (2) interfere with the ability of the promoter region to direct the transcription of the coding sequences, or (3) interfere with the ability of the corresponding RNA transcript to be translated into a protein.

A "polyadenylation signal sequence" (PAS) is a sequence known to those skilled in the art. The polyadenylation signal sequence typically comprises a conserved hexamer motif required for the polyadenylation of an mRNA, a U rich and/or GU rich sequence downstream of the hexamer motif, and a dinucleotide sequence that precedes the cleavage site for polyadenylation and is located between the U rich and/or GU rich sequence and the hexamer motif. The sequence can be recognized by cleavage and polyadenylation specificity factor (CPSF) within an RNA cleavage complex. The hexamer motif varies between eukaryotes but can be AATAAA or modifications thereof. CPSF is the central component of the 3' processing machinery for polyadenylated mRNAs and recognizes the PAS, thereby providing sequence specificity in both pre-mRNA cleavage and polyadenylation, and catalyzes pre-mRNA cleavage.

A "poly(A)-tail" (or "poly(A)-sequence") is an adenine nucleotide chain typically added to a mRNA molecule during RNA processing to increase the stability of the molecule and enable translation. This process, called polyadenylation, usually adds 100 to 250 adenines.

The terms "nucleic acid sequence" "nucleotide sequence", "polynucleotide" and "nucleic acid" can be used interchangeably herein to refer to one or more nucleotides, preferably deoxyribonucleic acid (DNA). These terms comprise DNA, ribonucleic acid (RNA), combinations thereof, and modified forms thereof. The term comprises genomic DNA, cDNA, mRNA, recombinantly produced and chemically synthesized molecules. In some preferred embodiments, a polynucleotide is DNA. In some embodiments, a polynucleotide is a mixture of DNA and RNA. A polynucleotide may be present as a single-stranded or double-stranded and linear or covalently circularly closed molecule. A polynucleotide can be isolated. The term "isolated polynucleotide " means, according to the present disclosure, that the polynucleotide (i) was amplified in vitro, for example via polymerase chain reaction (PCR) for DNA or in vitro transcription (using, e.g., an RNA polymerase) for RNA, (ii) was produced recombinantly by cloning, (iii) was purified, for example, by cleavage and separation by gel electrophoresis, or (iv) was synthesized, for example, by chemical synthesis.

The term "plasmid" refers to an extrachromosomal, mostly circular DNA capable of being expressed in a given cell. Plasmids can also be engineered by standard molecular biology techniques (Sambrook et al., Laboratory Manual, Cold Spring Harbor Laboratory Press, Cold Spring Harbor (1989), N.Y.). However, in embodiments relating to the stable cell expression system of the invention it is understood that the stable cell expression system may not comprise the plasmid as such but one or more sequences and genes of the plasmid, e.g., sequences of a plasmid stably integrated into the cell genome.

The terms "polypeptide," "peptide," and "protein" are used interchangeably herein to refer to polymers of amino acids.

The term "recombinant" when used in the context of a polynucleotide is a polynucleotide having nucleotide sequences that are not naturally joined together and can be made by artificially combining two otherwise separated segments of sequence. This artificial combination is often accomplished by chemical synthesis or, more commonly, by the artificial manipulation of isolated segments of nucleic acids, for example, by genetic engineering techniques. Recombinant polynucleotides include vectors comprising an amplified or assembled polynucleotide, which can be used to transform or transfect a suitable host cell. A host cell that comprises the recombinant polynucleotide is a "recombinant host cell." The polynucleotide is then expressed in the recombinant host cell to produce a "recombinant polypeptide." A recombinant polynucleotide can also comprise a non-coding function.

A "rep plasmid" is a plasmid comprising a replication protein coding sequence encoding a functional rep protein. The terms "rep plasmid" and "replication plasmid" can be used interchangeably herein.

A "rep cap plasmid" or "rep-cap plasmid" is a plasmid comprising a replication protein coding sequence encoding a functional rep protein and a capsid protein coding sequence encoding a functional cap protein. The terms "rep cap plasmid" and "replication capsid plasmid" can be used interchangeably herein.

"Recombinant AAV" (rAAV) and "AAV" are used interchangeably throughout the application.

As used herein, a "transgene" is a nucleic acid that is introduced into the cell, including but not limited to genes or nucleic acid having sequences which are not normally present in AAV, genes which are present but not normally transcribed and translated ("expressed") in an AAV genome, or any other gene or nucleic acid which one desires to position between the ITR sequences. A transgene may include one or more transcriptional regulatory sequences and any other nucleic acid, such as introns, that may be necessary for optimal expression of a selected nucleic acid. A transgene can be as few as a couple of nucleotides long, e.g., less than 50 or less than 30 nucleotides long, but can preferably be at least about 50, 100, 150, 200, 250, 300, 350, 400, 500, 600, 700, 800, 900, 1.000, 1.100, 1.200, 1.300, 1.400, 1.500, 1.600, 1.700, 1.800, 1.900, 2.000, 2.100, 2.200, 2.300, 2.400, 2.500, 2.600, 2.700, 2.800, 2.900, 3.000, 3.100, 3.200, 3.300, 3.400, 3.500, 3.600, 3.700, 3.800, 3.900, 4.000, 4.100, 4.200, 4.300, 4.400, 4.500, or 4.600 nucleotides (nt) long. A transgene can comprise coding or non-coding sequences.

As used herein, the term "variant" refers to a molecule, such as a gene or protein, that shares one or more particular structural features, elements, components, or moieties with a reference molecule. A variant therefore is a molecule, such as a gene or protein, that shares one or more particular structural features, elements, components, or moieties with a reference molecule. A variant can be a gene that shares one or more particular structural features, elements, components, or moieties with a reference gene. A variant can be a protein that shares one or more particular structural features, elements, components, or moieties with a reference protein. Typically, a "variant" has significant structural similarity with the reference molecule, for example sharing a core or consensus structure, but also differs in certain discrete ways. In some embodiments, a variant can be an artificial variant that has been altered by human intervention. In some embodiments, an artificial variant is a molecule that can be generated from the reference molecule, e.g., by genetic engineering and/or chemical manipulation of the reference molecule. In some embodiments, an artificial variant is a molecule that can be generated through performance of a synthetic process substantially similar to (e.g., sharing a plurality of steps with) one that generates the reference molecule. In some embodiments, an artificial variant is or can be generated through performance of a synthetic process different from that used to generate the reference molecule.

As used herein, the terms "virus vector," "viral vector," and "gene delivery vector" refer to a virus particle that functions as a nucleic acid delivery vehicle, and which comprises a nucleic acid molecule packaged within a viral capsid. Exemplary virus vectors include adeno-associated virus (AAV) vectors.

As used herein, the term "transduction efficiency" refers to the ability of a viral vector or viral particle, preferably an rAAV particle, to transduce a target cell. As used herein, the term "potency" refers to the ability of a viral vector or viral particle, preferably an rAAV particle, to transduce a target cell and express a transgene in the target cell. Potency can be represented in transducing units (e.g., TU per volume). Transducing unit as used herein refers to the number of functional viral vectors or viral particles, preferably rAAV particles, in a solution that are capable of transducing a target cell and expressing a transgene in the target cell. The skilled person knows how to determine the potency of a viral vector or a viral particle. For example, potency can be determined according to the methods described in Green and Kelvin, "Analytical methods to characterize recombinant adeno-associated virus vectors and the benefit of standardization and reference materials ", Curr Opin Biotechnol. 2021 Oct; 71: 65-76, doi: 10.1016/j.copbio.2021.06.025.

The terms "wildtype" and "native" as used herein are synonymous and refer to polynucleotide sequences or genes present in the genome of a strain/serotype of AAV or adenovirus, or to proteins encoded by genes present in the genome of a strain/serotype of AAV or adenovirus. Preferably, the wildtype strain/serotype of AAV can refer to the AAV2 genome shown in the GenBank accession code J01901.1.

Recitation of ranges of values herein is merely intended to serve as a shorthand method of referring individually to each separate value falling within the range. Unless otherwise indicated herein, each individual value is incorporated into the specification as if it was individually recited herein. All methods described herein can be performed in any suitable order unless otherwise indicated herein or otherwise clearly contradicted by context. The use of any and all examples, or exemplary language (e.g., "such as"), provided herein is intended merely to better illustrate the invention and does not pose a limitation on the scope of the invention otherwise claimed. No language in the specification should be construed as indicating any non-claimed element essential to the practice of the invention.

References to "one embodiment," "an embodiment," "example embodiment," "some embodiments," "certain embodiments," "various embodiments," etc., indicate that the embodiment(s) of the disclosed technology so described may include a particular feature, structure, or characteristic, but not every embodiment necessarily includes the particular feature, structure, or characteristic.

All patents, patent applications, and other publications cited in this application are incorporated by reference in the entirety for all purposes.

### The rep plasmid of the invention

The present invention provides a rep plasmid comprising:
(i) at least one long adeno-associated virus replication protein coding sequence encoding at least one long functional rep protein;
(ii) at least one short adeno-associated virus replication protein coding sequence encoding at least one short functional rep protein; and
(iii) at least one heterologous element, wherein following transcription the at least one heterologous element results in a ratio of long adeno-associated virus rep mRNA to short adeno-associated virus rep mRNA of about 1:5 to about 1:5000.

Without wishing to be bound by theory, rAAV vector production is contemplated to be improved by optimizing the ratio of long rep to short rep proteins. In particular, improving the ratio of long to short rep proteins in a rAAV production cell, (e.g., a mammalian cell) is considered to be beneficial for efficient rAAV production. The rep plasmid according to the present invention enables tailoring of the ratio of long to short rep proteins by improving the ratio of long rep protein mRNA to short rep protein mRNA. It has been surprisingly found that low concentrations of long rep proteins relative to the concentration of short rep proteins result in improved rAAV vector production. As demonstrated in the examples (e.g., examples 2-3), cells transfected with the rep plasmid of the invention achieved an improved potency. Therefore, rAAV particles produced using the rep plasmid of the invention provided an increased potency. The rep plasmid of the invention is therefore particularly suitable for producing rAAVs for gene therapy applications, wherein a high titer of functional rAAV vectors is required for efficient and/or effective treatment.

According to the present invention, the rep plasmid comprises at least one heterologous element which provides an improved ratio of long rep to short rep proteins. In some embodiments, the heterologous element is selected from a heterologous promoter, a heterologous polyadenylation signal sequence, and a heterologous nuclear export enhancer (e.g., a woodchuck post-transcriptional element). In some embodiments, the heterologous element is a destabilization domain, RNAi target, an enhancer region, a repressor region, a modified STOP codon, a modified START codon, or an UTR secondary structure. In some embodiments, the heterologous element influences the splicing system. In some embodiments, the heterologous element is selected from at least one heterologous promoter and at least one heterologous polyadenylation signal sequence. In some preferred embodiments, the heterologous promoter is operatively linked to the long adeno-associated virus rep protein coding sequence.

Without wishing to be bound by theory, it is contemplated that the ratio of the long rep to short rep proteins, i.e., the ratio after transcription and/or the ratio after translation, can be influenced by the heterologous promoter operatively linked to the long adeno-associated rep protein coding sequence. In some embodiments, the heterologous promoter operatively linked to the long adeno-associated rep protein coding sequence is characterized by a decreased promoter strength as compared to the native promoter of the short adeno-associated virus rep protein coding sequence. In the context of decreased promoter strength of the heterologous promoter operatively linked to the long adeno-associated rep protein coding sequence, the native promoter of the short adeno-associated virus rep protein coding sequence is a p19 promoter which can be selected from different AAV serotypes. Promoter strength as used herein refers to the ability of a promoter to induce transcription of an RNA transcript from a DNA template. Promoter strength may be determined based on RNA levels after transcription or based on protein levels after translation. The skilled person can readily determine the strength of a promoter, e.g., by using PCR-based techniques to determine RNA transcript levels (e.g., by digitalPCR or real-time quantitative PCR) or by using an expression assay to determine protein levels after translation. Reduced or increased promoter strength as used herein refers to the strength of a first promoter compared to the strength of a second promoter. In some embodiments, the second promoter can be a reference promoter. In some embodiments, the first promoter can be a heterologous promoter as described herein. Whether the strength of the first promoter is decreased or increased compared to the second promoter can be determined by the skilled person by using a test plasmid comprising the first promoter whose strength is to be determined, and a reference plasmid comprising the second promoter, e.g., the reference promoter. According to the present disclosure, the strength of the heterologous promoter or the first promoter is considered to be reduced when the promoter has 95% or less, 90% or less, 80% or less, 70% or less, 60% or less, 50% or less, 40% or less, 30% or less, 20% or less, 10% or less, 9% or less, 8% or less, 7% or less, 6% or less, 5% or less, 4% or less, 3% or less, 2% or less, 1% or less, 0.5% or less, or 0.1% or less of the strength of the second promoter or the reference promoter. In some embodiments, the strength of the heterologous promoter or the first promoter is considered to be reduced when the promoter has 40% or less, 30% or less, 25% or less, 20% or less, 15% or less, 10% or less, 9% or less, 8% or less, 7% or less, 6% or less, 5% or less, 4% or less, 3% or less, 2% or less, 1% or less, 0.5% or less, or 0.1% or less of the strength of the second promoter or reference promoter.

In some embodiments, the heterologous promoter operatively linked to the long adeno-associated rep protein coding sequence has 95% or less, 90% or less, 80% or less, 70% or less, 60% or less, 50% or less, 40% or less, 30% or less, 20% or less, 10% or less, 9% or less, 8% or less, 7% or less, 6% or less, 5% or less, 4% or less, 3% or less, 2% or less, 1% or less, 0.5% or less, or 0.1% or less of the strength of the native promoter (i.e., p19 promoter, preferably AAV2 p19 promoter) operatively linked to the short adeno-associated virus rep protein coding sequence.

In some preferred embodiments, the heterologous promoter operatively linked to the long adeno-associated rep protein coding sequence has 40% or less, 30% or less, 25% or less, 20% or less, 15% or less, 10% or less, 9% or less, 8% or less, 7% or less, 6% or less, 5% or less, 4% or less, 3% or less, 2% or less, 1% or less, 0.5% or less, or 0.1% or less of the strength of the native promoter (i.e., p 19 promoter, preferably AAV2 p 19 promoter) operatively linked to the short adeno-associated virus rep protein coding sequence.

In some preferred embodiments, the heterologous promoter operatively linked to the long adeno-associated rep protein coding sequence has 25% or less, 20% or less, 15% or less, 10% or less, 9% or less, 8% or less, 7% or less, 6% or less, 5% or less, 4% or less, 3% or less, 2% or less, 1% or less, 0.5% or less, or 0.1% or less of the strength of the native promoter (i.e., p19 promoter, preferably AAV2 p19 promoter) operatively linked to the short adeno-associated virus rep protein coding sequence.

In some preferred embodiments, the heterologous promoter is selected from a mouse mammary tumor virus promoter (pMMTV), a glycoprotein hormones alpha polypeptide promoter (pCGA), a dynein axonemal heavy chain 17 promoter (pDNAH17), and an anti-mullerian hormone promoter (pAMH). In some preferred embodiments, the heterologous promoter is operatively linked to the long adeno-associated rep protein coding sequence and is selected from a MMTV promoter, a CGA promoter, a DNAH17 promoter, and an AMH promoter. In some embodiments, the heterologous promoter operatively linked to the long adeno-associated rep protein coding sequence is a MMTV promoter. In some embodiments, the MMTV promoter comprises or consists of a sequence set forth in SEQ ID NO: 13 or a sequence having at least 60%, at least 70%, at least 80%, or at least 90% identity to said SEQ ID NO. In some embodiments, the heterologous promoter operatively linked to the long adeno-associated rep protein coding sequence is a CGA promoter. In some embodiments, the CGA promoter comprises or consists of a sequence set forth in SEQ ID NO: 14 or a sequence having at least 60%, at least 70%, at least 80%, or at least 90% identity to said SEQ ID NO. In some embodiments, the heterologous promoter operatively linked to the long adeno-associated rep protein coding sequence is a DNAH17 promoter. In some embodiments, the DNAH17 promoter comprises or consists of a sequence set forth in SEQ ID NO: 15 or a sequence having at least 60%, at least 70%, at least 80%, or at least 90% identity to said SEQ ID NO. In some embodiments, the heterologous promoter operatively linked to the long adeno-associated rep protein coding sequence is an AMH promoter. In some embodiments, the AMH promoter comprises or consists of a sequence set forth in SEQ ID NO: 16 or a sequence having at least 60%, at least 70%, at least 80%, or at least 90% identity to said SEQ ID NO.

In some embodiments, the heterologous promoter is selected from an artificial variant of a native promoter present in the AAV genome. In some embodiments, the heterologous promoter is selected from a variant of the native p5 promoter, wherein the artificial p5 promoter variant is characterized by a reduced promoter strength compared to the native p5 promoter. In some embodiments, the long adeno-associated rep protein coding sequence is operatively linked to an artificial variant of the native p5 promoter, wherein the p5 promoter variant is characterized by a reduced promoter strength compared to the native p5 promoter. The skilled person can readily determine whether the strength of the p5 promoter variant is reduced compared to the native p5 promoter, e.g., by using PCR-based techniques to determine RNA transcript levels (e.g., by digitalPCR or real-time quantitative PCR) or by using an expression assay to determine protein levels after translation. Reduced promoter strength of the p5 promoter variant as used herein refers to the strength of the p5 promoter variant compared to the strength of the native p5 promoter. Whether the strength of the p5 promoter variant is decreased compared to the native p5 promoter can be determined by the skilled person by using a test plasmid comprising the p5 promoter variant whose strength is to be determined, and a reference plasmid comprising the native p5 promoter. According to the present disclosure, the strength of the p5 promoter variant is considered to be reduced when the promoter has 95% or less, 90% or less, 80% or less, 70% or less, 60% or less, 50% or less, 40% or less, 30% or less, 20% or less, 10% or less, 9% or less, 8% or less, 7% or less, 6% or less, 5% or less, 4% or less, 3% or less, 2% or less, 1% or less, 0.5% or less, or 0.1% or less of the strength of the native p5 promoter. In some embodiments, the strength of the p5 promoter variant is considered to be reduced when the promoter has 40% or less, 30% or less, 25% or less, 20% or less, 15% or less, 10% or less, 9% or less, 8% or less, 7% or less, 6% or less, 5% or less, 4% or less, 3% or less, 2% or less, 1% or less, 0.5% or less, or 0.1% or less of the strength of the native p5 promoter.

In some embodiments, the rep plasmid comprises one or more polyadenylation signal sequences.

In some embodiments, the polyadenylation signal sequence is a native polyadenylation signal sequence, i.e., a polyadenylation sequence comprised in the wildtype AAV genome. In some embodiments, the polyadenylation signal sequence is a heterologous polyadenylation signal sequence, i.e., a polyadenylation signal sequence which is not comprised in the wild type AAV genome.

In some embodiments, the heterologous element is a heterologous polyadenylation signal sequence.

In some embodiments, the native or heterologous polyadenylation signal sequence can be a sequence comprising AATAAA or a modified sequence thereof. The modified sequence of AATAAA can be a sequence in which one or two nucleic acids are deleted, substituted, inserted and/or added. Other polyadenylation signal sequences are known, for example, ATTAAA, AGTAAA, TATAAA, CATAAA, GATAAA, AATATA, AATACA, AATAGA, AAAAAG, and ACTAAA, and can be used in context of the present disclosure. For clarity, the plasmids disclosed herein can comprise sequences (or elements) that can control or influence polyadenylation such as U/GU-rich sequences, which can be located upstream or downstream of the AATAAA or a modified sequence, e.g., separated by several (random) nucleotides (5, 10, 20, 30, 40, 50 60, 70, 80, 90, 100, 1000, 2000, or more nucleotides).

Preferably, the heterologous polyadenylation signal sequence used in context of the disclosure is a strong polyadenylation signal sequence. Without wishing to be bound by theory, a strong polyadenylation signal is contemplated to improve the nuclear export of long and/or short rep protein mRNA, leading to increased translation of long and/or short rep protein mRNA and therefore to higher titers of functional rAAV particles, i.e., rAAV particles with high potency. The strength of a given polyadenylation signal sequence as described herein refers to the ability of the polyadenylation signal sequence to terminate transcription and initiate polyadenylation of the mRNA's 3' end. Polyadenylation signal sequence strength can be determined based on the degree of polyadenylation of a given mRNA. The skilled person can therefore readily determine the strength of a polyadenylation signal sequence by using, e.g., PCR-based methods (e.g., digitalPCR, real-time quantitative PCR, RNA-Seq), Northern Blotting, and Microarray methods to determine mRNA polyadenylation levels. For example, polyadenylation signal sequence strength can be determined by the methods described in Hoque et al., "Analysis of alternative cleavage and polyadenylation by 3' region extraction and deep sequencing", Nature Methods volume 10, pages133-139 (2013), doi: 10.1038/nmeth.2288 and Proudfoot, "Ending the message: poly(A) signals then and now", Genes Dev. 2011 Sep 1; 25(17): 1770-1782, doi: 10.1101/gad.17268411.

Non-limiting examples of heterologous strong polyadenylation signal sequences are SV40 and rabbit β-globin poly(A) signal sequences.

In some embodiments, the heterologous polyadenylation signal sequence is capable of forming a proper poly(A) sequence at the RNA's 3'end. Examples of heterologous polyadenylation signal sequences are, without being limited, an adenovirus L3 poly(A) signal sequence, HSV TK poly(A) signal sequence, hGH poly(A) signal sequence, spA poly(A) signal sequence, rabbit gbpA poly(A) signal sequence, sNRP1 poly(A) signal sequence, bGH poly(A) signal sequence, synthetic poly(A) signal sequence, mouse β-globin poly(A) signal sequence, rabbit β-globin poly(A) signal sequence, H4-based poly(A) signal sequence, and SV40 poly(A) signal sequence. In some preferred embodiments, the heterologous polyadenylation signal sequence is a rabbit β-globin poly(A) signal sequence. In some preferred embodiments, the heterologous polyadenylation signal sequence comprises or consists of a sequence set forth in SEQ ID NO: 23 or a sequence having at least 60%, at least 70%, at least 80%, or at least 90% identity to said SEQ ID NO.

In some embodiments, the heterologous polyadenylation signal sequence is located downstream of the rep protein coding sequence. In some embodiments, given by way of non- limiting example, downstream of the rep protein coding sequence can mean that the 5' end of the heterologous polyadenylation signal sequence can be located e.g., within 3000 or less nucleotides, 2000 or less nucleotides, 1000 or less nucleotides, 500 or less nucleotides, 100 or less nucleotides, 75 or less nucleotides, 50 or less nucleotides, 25 or less nucleotides, 20 or less nucleotides, 15 or less nucleotides, 10 or less nucleotides, or 5 or less nucleotides downstream of the rep protein coding sequence. It is understood that a skilled person can identify a suitable location of the polyadenylation signal sequence.

In some embodiments, the heterologous polyadenylation signal sequence is located downstream of the cap protein coding sequence. In some embodiments given, by way of non- limiting example, downstream of the cap protein coding sequence can mean that the 5' end of the heterologous polyadenylation signal sequence can be located e.g., within 3000 or less nucleotides, 2000 or less nucleotides, 1000 or less nucleotides, 500 or less nucleotides, 100 or less nucleotides, 75 or less nucleotides, 50 or less nucleotides, 25 or less nucleotides, 20 or less nucleotides, 15 or less nucleotides, 10 or less nucleotides, or 5 or less nucleotides downstream of the cap protein coding sequence.

In some embodiments, the rep plasmid comprises one or more polyadenylation signal sequences. In some embodiments, the rep plasmid comprises at least two polyadenylation signal sequences. In some embodiments, the rep plasmid comprises two polyadenylation signal sequences. In such embodiments, the (at least) two polyadenylation signal sequences can either be the same polyadenylation signal sequence or can be different polyadenylation signal sequences. For example, the rep plasmid can comprise a first native polyadenylation signal sequence and a second strong heterologous polyadenylation signal sequence, e.g., a rabbit β-globin poly(A) signal sequences as shown in SEQ ID NO: 23.

In some embodiments, a p5 promoter or a variant thereof is located upstream of the heterologous polyadenylation signal sequence. In some embodiments, the p5 promoter or variant thereof comprises or consists of a sequence set forth in SEQ ID NO: 17 or a sequence having at least 60%, at least 70%, at least 80%, or at least 90% identity to said SEQ ID NO. The rep plasmid can e.g., comprise the p5 promoter or variant thereof immediately upstream of the heterologous polyadenylation signal sequence. In some embodiments given by way of non- limiting example, immediately upstream means that the 3' end of the p5 promoter or variant thereof can be located e.g., within 1000 or less nucleotides, 500 or less nucleotides, 100 or less nucleotides, 75 or less nucleotides, 50 or less nucleotides, 25 or less nucleotides, 20 or less nucleotides, 15 or less nucleotides, 10 or less nucleotides or 5 or less nucleotides upstream of the heterologous polyadenylation signal sequence. It is understood that a skilled person can identify a suitable location of any promoter disclosed herein. As a further non-limiting example, the p5 promoter or a variant thereof can be located upstream of the heterologous polyadenylation signal sequence without a gene (e.g., promoter, RNA polymerase II-driven gene, and polyadenylation signal sequence) located in the same orientation between the 3' end of the p5 promoter and the 5' end of the heterologous polyadenylation signal sequence. In the context of this example, the term "gene" refers to a polynucleotide sequence which encodes a functional polypeptide, e.g., a functional rep protein.

In some embodiments, a rep-binding element (RBE) derived from a p5 promoter is located upstream of the heterologous polyadenylation signal sequence. The rep plasmid can e.g., comprise the RBE derived from the p5 promoter immediately upstream of the heterologous polyadenylation signal sequence. In some embodiments given by way of non-limiting example, immediately upstream means that the 3' end of the RBE can be located e.g., within 1000 or less nucleotides, 500 or less nucleotides, 100 or less nucleotides, 75 or less nucleotides, 50 or less nucleotides, 25 or less nucleotides, 20 or less nucleotides, 15 or less nucleotides, 10 or less nucleotides or 5 or less nucleotides upstream of the heterologous polyadenylation signal sequence. In some embodiments, the RBE derived from the p5 promoter has the sequence shown in SEQ ID NO: 12. It is understood that a skilled person can identify a suitable location of the RBE disclosed herein. As a further non-limiting example, the RBE derived from the p5 promoter can located upstream of the heterologous polyadenylation signal sequence without a gene (e.g., promoter, RNA polymerase II-driven gene, and polyadenylation signal sequence) located in the same orientation between the 3' end of the RBE and the 5' end of the heterologous polyadenylation signal sequence. In the context of this example, the term "gene" refers to a polynucleotide sequence which encodes a functional polypeptide, e.g., a functional rep protein.

In some embodiments, a rep-binding element (RBE) derived from a p5 promoter is located downstream of the heterologous polyadenylation signal sequence. The rep plasmid can e.g., comprise the RBE derived from the p5 promoter immediately downstream of the heterologous polyadenylation signal sequence. In some embodiments, given by way of non-limiting example, immediately downstream means that the 5' end of the RBE can be located e.g., within 1500 or less nucleotides, 1000 or less nucleotides, 500 or less nucleotides, 100 or less nucleotides, 75 or less nucleotides, 50 or less nucleotides, 25 or less nucleotides, 20 or less nucleotides, 15 or less nucleotides, 10 or less nucleotides or 5 or less nucleotides downstream of the heterologous polyadenylation signal sequence.In some embodiments, the rep plasmid can comprise one or more rep-binding elements (RBE). A RBE can function as binding site for rep68 and/or rep78. In some embodiments, the one or more RBEs function as binding site for rep68 or rep78. In some embodiments, the one or more RBEs function as binding site for rep68 and rep78. In some embodiments, the one or more RBEs function as binding site for rep68. In some embodiments, the one or more RBEs function as binding site for rep78. In some embodiments, the RBE is derived from a non-ITR sequence, such as an AAV promoter sequence. In some embodiments, the RBE is derived from a p5 promoter. The RBE may preferably comprise or consist of the sequence shown in SEQ ID NO: 12 or a sequence having at least 60%, at least 70%, at least 80%, or at least 90% identity to said SEQ ID NO. The RBE may comprise or consist of the sequence shown in SEQ ID NO: 26 or a sequence having at least 60%, at least 70%, at least 80%, or at least 90% identity to said SEQ ID NO.

In some embodiments, the rep plasmid of the invention can comprise more than one heterologous element. For example, the rep-plasmid can comprise a heterologous promoter operatively linked to the long adeno-associated virus rep protein coding sequence and a heterologous strong polyadenylation signal sequence.

The rep plasmid of the invention can further comprise a promoter that controls transcription of the short rep protein coding sequence. In some embodiments, the short adeno-associated virus rep protein coding sequence is operatively linked to a promoter. In some preferred embodiments the short adeno-associated virus rep protein coding sequence is operatively linked to a native or heterologous promoter. In some embodiments, the promoter operatively linked to the short adeno-associated virus rep protein coding sequence is selected from a p19 promoter, an elongation factor 1A promoter (pEF1A), a transmembrane BAX inhibitor motif containing 6 promoter (pTMBIM6), and a glycerinaldehyd-3-phosphat-dehydrogenase promoter (pGAPDH). In some embodiments, the promoter operatively linked to the short adeno-associated virus rep protein coding sequence is a p19 promoter. In some embodiments, the p19 promoter comprises or consists of a sequence set forth in SEQ ID NO: 18 or a sequence having at least 60%, at least 70%, at least 80%, or at least 90% identity to said SEQ ID NO. In some embodiments, the promoter operatively linked to the short adeno-associated virus rep protein coding sequence is a EF1A promoter. In some embodiments, the EF1A promoter comprises or consists of a sequence set forth in SEQ ID NO: 19 or a sequence having at least 60%, at least 70%, at least 80%, or at least 90% identity to said SEQ ID NO. In some embodiments, the promoter operatively linked to the short adeno-associated virus rep protein coding sequence is a TMBIM6 promoter. In some embodiments, the TMBIM6 promoter comprises or consists of a sequence set forth in SEQ ID NO: 20 or a sequence having at least 60%, at least 70%, at least 80%, or at least 90% identity to said SEQ ID NO. In some embodiments, the promoter operatively linked to the short adeno-associated virus rep protein coding sequence is a GAPDH promoter. In some embodiments, the GAPDH promoter or variant thereof comprises or consists of a sequence set forth in SEQ ID NO: 21 or a sequence having at least 60%, at least 70%, at least 80%, or at least 90% identity to said SEQ ID NO.

In some embodiments, the rep plasmid comprises a heterologous promoter operatively linked to the long adeno-associated virus rep protein coding sequence and a native or heterologous promoter operatively linked to the short adeno-associated virus rep protein coding sequence. In some embodiments, the rep plasmid comprises a heterologous promoter operatively linked to the long adeno-associated virus rep protein coding sequence and a native promoter operatively linked to the short adeno-associated virus rep protein coding sequence. In some embodiments, the rep plasmid comprises a heterologous promoter operatively linked to the long adeno-associated virus rep protein coding sequence and a heterologous promoter operatively linked to the short adeno-associated virus rep protein coding sequence. For example, the rep plasmid can comprise a MMTV promoter operatively linked to the long adeno-associated virus rep protein coding sequence and a p19 promoter operatively linked to the short adeno-associated virus rep protein coding sequence.

In some embodiments, the rep plasmid disclosed herein comprises a promoter selected from a constitutive, inducible, or repressible promoter. In some embodiments, the rep plasmid disclosed herein comprises promoters selected from a constitutive, inducible, or repressible promoters or any combination thereof. For example, the long adeno-associated virus rep protein coding sequence can be operatively linked to a constitutive, inducible, or repressible promoter. In some embodiments, the short adeno-associated virus rep protein coding sequence can be operatively linked to a constitutive, inducible, or repressible promoter. Examples of inducible and/or repressible promoters include, but are not limited to, p5, p19, pCGA, pDNAH17, and pAMH. pMMTV is a non-limiting example of a constitutive promoter.

The ratio of long rep to short rep proteins is optimized by the heterologous element of the rep plasmid disclosed herein. In some preferred embodiments, the heterologous element is a heterologous promoter. In some preferred embodiments, the heterologous promoter is operatively linked to the long adeno-associated virus rep protein coding sequence. In some preferred embodiments, the heterologous element is a heterologous polyadenylation signal sequence. In some preferred embodiments, the ratio of long rep to short rep proteins is optimized by the heterologous promoter operatively linked to the long adeno-associated virus rep protein coding sequence and by a heterologous polyadenylation signal as disclosed herein. The skilled person knows how to determine ratios of different proteins after expression, e.g., using an expression assay. For example, the ratio of long to short rep proteins can be determined by Western blot or Enzyme-linked Immunosorbent Assay (ELISA) methods. In the context of the present disclosure, the ratio of long rep proteins to short rep proteins refers to the ratio of total long rep proteins to total short rep proteins, e.g., the ratio of total long rep proteins to total short rep proteins present in the producer cell. For example, the ratio of long rep proteins to short rep proteins can be the ratio of rep68 to rep52. The ratio of long rep proteins to short rep proteins can be the ratio of rep68 and rep78 to rep40 and rep52.

In some preferred embodiments, the ratio of long rep mRNA to short rep mRNA refers to the ratio of total long rep mRNA to total short rep mRNA, e.g., the ratio of total long rep mRNA to total short rep mRNA present in the producer cell. For example, the ratio of long rep mRNA to short rep mRNA can be the ratio of rep68 mRNA to rep52 mRNA. The ratio of long rep protein mRNA to short rep protein mRNA can be the ratio of rep68 mRNA and rep78 mRNA to rep40 mRNA and rep52 mRNA.

As disclosed herein for the rep plasmid, following transcription the at least one heterologous element results in a ratio of long adeno-associated virus rep mRNA to short adeno-associated virus rep mRNA of about 1:5 to about 1:5000.

In some embodiments, the ratio of long adeno-associated virus replication proteins to short adeno-associated virus replication proteins is
(i) about 1:5 to about 1:300, preferably about 1:5 to about 1:250, more preferably about 1:5 to about 1:200, even more preferably about 1:5 to about 1:150, and most preferably about 1:5 to about 1:100, optionally, wherein the long adeno-associated virus rep protein level is high; and/or
(ii) about 1:5 to about 1:5000, preferably about 1:20 to about 1:2500, more preferably about 1:50 to about 1:2000, and most preferably about 1: 100 to about 1: 1500, optionally, wherein the long adeno-associated virus rep protein level is low.

In some embodiments, the ratio of long adeno-associated virus replication proteins to short adeno-associated virus replication proteins is
(i) about 1:5 to about 1:300, preferably about 1:5 to about 1:250, more preferably about 1:5 to about 1:200, even more preferably about 1:5 to about 1:150, and most preferably about 1:5 to about 1:100, wherein the long adeno-associated virus rep protein level is high; or
(ii) about 1:5 to about 1:5000, preferably about 1:20 to about 1:2500, more preferably about 1:50 to about 1:2000, and most preferably about 1: 100 to about 1: 1500, wherein the long adeno-associated virus rep protein level is low.

In some embodiments, the ratio between long and short rep proteins is about 1:5 to about 1:300. In some preferred embodiments, the ratio between long and short rep proteins is about 1:5 to about 1:250. In some more preferred embodiments, the ratio between long and short rep proteins is about 1:5 to about 1:200. In some even more preferred embodiments, the ratio between long and short rep proteins is about 1:5 to about 1:150. In some most preferred embodiments, the ratio between long and short rep proteins is about 1:5 to about 1:100. In some such embodiments, the long adeno-associated virus rep protein level may be high. Specifically, it was found that for a high level of long adeno-associated virus rep protein, the ratio between long and short rep proteins is about 1:5 or higher. Without being bound to theory, it is hypothesized that the long rep protein has the same domains as the short rep protein in addition to DNA binding domain(s), such that the long rep protein may take over some of the short rep protein function(s).

In some embodiments, the ratio between long and short rep proteins is about 1:15 to about 1:200, about 1:20 to about 1:200, about 1:20 to about 1:150, about 1:25 to about 1:150, about 1:25 to about 1:125, about 1:25 to about 100, about 1:30 to about 1:95, about 1:35 to about 1:90, about 1:40 to about 1:85, about 1:45 to about 1:80, about 1:50 to about 1:75 or about 1:55 to about 1:70. In some embodiments, the ratio between long and short rep proteins is about 1:20 to about 1:150, about 1:25 to about 1:150, about 1:25 to about 1:125, about 1:25 to about 100, about 1:30 to about 1:95, about 1:35 to about 1:90, about 1:40 to about 1:85, about 1:45 to about 1:80, about 1:50 to about 1:75 or about 1:55 to about 1:70. In some embodiments, the ratio between long and short rep proteins is about 1:5 to about 1:100, about 1:5 to about 1:95, about 1:5 to about 1:90, about 1:5 to about 1:85, about 1:5 to about 1:80, about 1:5 to about 1:75, or about 1:5 to about 1:70. Optionally, in such embodiment the long adeno-associated virus rep protein level is high.

In some embodiments, the ratio between long and short rep proteins is about 1:300, about 1:275, about 1:250, about 1:200, about 1:175, about 1:150, about 1:125, about 1:100, about 1:90, about 1:80, about 1:70, about 1:60, about 1:50, about 1:45, about 1:40, about 1:35, about 1:30, about 1:25, about 1:20, about 1:15, about 1:10, about 1:9, about 1:8, about 1:7, about 1:6, or about 1:5. In some embodiments, the ratio between long and short rep proteins is about 1:100, about 1:90, about 1:80, about 1:70, about 1:60, about 1:50, about 1:45, about 1:40, about 1:35, about 1:30, about 1:25, about 1:20, about 1:15, about 1:10, about 1:9, about 1:8, about 1:7, about 1:6, or about 1:5. Optionally, in such embodiment the long adeno-associated virus rep protein level is high.

In some embodiments, the ratio between long and short rep proteins is about 1:5 to about 1:5000. In some preferred embodiments, the ratio between long and short rep proteins is about 1:20 to about 1:2500. In some more preferred embodiments, the ratio between long and short rep proteins is about 1:50 to about 1:2000. In some most preferred embodiments, the ratio between long and short rep proteins is about 1:100 to about 1:1500. In such embodiments, the long adeno-associated virus rep protein level may be low. Specifically, it was found that for a low level of long adeno-associated virus rep protein, the ratio between long and short rep proteins is about 1:5 or higher, preferably about 1:100 or higher. Such high ratios advantageously increase the potency.

In some embodiments, the ratio between long and short rep proteins is about 1:20 to about 1:4000, about 1:25 to about 1:3500, about 1:30 to about 1:3000, about 1:35 to about 1:2500, about 1:40 to about 1:2400, about 1:45 to about 2300, about 1:50 to about 1:2200, about 1:55 to about 1:2100, about 1:60 to about 1:2000, about 1:65 to about 1:1900, about 1:70 to about 1:1800 or about 1:75 to about 1:1700. In some embodiments, the ratio between long and short rep proteins is about 1: 100 to about 1: 1500. Optionally, in such embodiment the long adeno-associated virus rep protein level is low.

In some embodiments, the ratio between long and short rep proteins is about 1:100 or higher, about 1:200 or higher, about 1:300 or higher, about 1:400 or higher, about 1:500 or higher, about 1:600 or higher, about 1:700 or higher, about 1:800 or higher, about 1:900 or higher, or about 1: 1000 or higher. Optionally, in such embodiments, the long adeno-associated virus rep protein level is low.

In some embodiments, after transcription of the rep protein coding sequence, the ratio of long adeno-associated virus replication protein mRNA to short adeno-associated virus replication protein mRNA is
(i) about 1:5 to about 1:300, preferably about 1:5 to about 1:250, more preferably about 1:5 to about 1:200, even more preferably about 1:5 to about 1:150, and most preferably about 1:5 to about 1:100, optionally, wherein the long adeno-associated virus rep protein mRNA level is high; and/or
(ii) about 1:5 to about 1:5000, preferably about 1:20 to about 1:2500, more preferably about 1:50 to about 1:2000, and most preferably about 1: 100 to about 1: 1500, optionally, wherein the long adeno-associated virus rep protein mRNA level is low.

In some embodiments, after transcription of the rep protein coding sequence, the ratio of long adeno-associated virus replication protein mRNA to short adeno-associated virus replication protein mRNA is
(i) about 1:5 to about 1:300, preferably about 1:5 to about 1:250, more preferably about 1:5 to about 1:200, even more preferably about 1:5 to about 1:150, and most preferably about 1:5 to about 1:100, wherein the long adeno-associated virus rep protein mRNA level is high; or
(ii) about 1:5 to about 1:5000, preferably about 1:20 to about 1:2500, more preferably about 1:50 to about 1:2000, and most preferably about 1:100 to about 1:1500, wherein the long adeno-associated virus rep protein mRNA level is low.

The rep plasmid according to embodiment 1, wherein the ratio of long adeno-associated virus replication mRNA to short adeno-associated virus replication mRNA is
(i) about 1:5 to about 1:300, preferably about 1:5 to about 1:250, more preferably about 1:5 to about 1:200, even more preferably about 1:5 to about 1:150, and most preferably about 1:5 to about 1:100, optionally, wherein the long adeno-associated virus rep protein mRNA level is high; and/or
(ii) about 1:5 to about 1:5000, preferably about 1:20 to about 1:2500, more preferably about 1:50 to about 1:2000, and most preferably about 1:100 to about 1:1500, optionally, wherein the long adeno-associated virus rep protein mRNA level is low.

The rep plasmid according to embodiment 1, wherein the ratio of long adeno-associated virus replication mRNA to short adeno-associated virus replication mRNA is
(i) about 1:5 to about 1:300, preferably about 1:5 to about 1:250, more preferably about 1:5 to about 1:200, even more preferably about 1:5 to about 1:150, and most preferably about 1:5 to about 1:100, wherein the long adeno-associated virus rep protein mRNA level is high; or
(ii) about 1:5 to about 1:5000, preferably about 1:20 to about 1:2500, more preferably about 1:50 to about 1:2000, and most preferably about 1:100 to about 1:1500, wherein the long adeno-associated virus rep protein mRNA level is low.

In some embodiments, after transcription of the rep protein coding sequence, the ratio of long adeno-associated virus rep protein mRNA to short adeno-associated virus rep protein mRNA is about 1:5 to about 1:300. In some preferred embodiments, after transcription of the rep protein coding sequence, the ratio of long adeno-associated virus rep mRNA to short adeno-associated virus rep mRNA is about 1:5 to about 1:250. In some more preferred embodiments, after transcription of the rep protein coding sequence, the ratio of long adeno-associated virus rep mRNA to short adeno-associated virus rep mRNA is about 1:5 to about 1:200. In some even more preferred embodiments, after transcription of the rep protein coding sequence, the ratio of long adeno-associated virus rep mRNA to short adeno-associated virus rep mRNA is about 1:5 to about 1:150. In some most preferred embodiments, after transcription of the rep protein coding sequence, the ratio of long adeno-associated virus rep mRNA to short adeno-associated virus rep mRNA is about 1:5 to about 1:100. In such embodiments, the long adeno-associated virus rep protein mRNA level may be high. Specifically, it was found that for a high level of long adeno-associated virus rep protein mRNA, the ratio between long and short rep proteins is about 1:5 or higher. Without being bound to theory, it is hypothesized that the long rep protein has the same domains as the short rep protein in addition to DNA binding domain(s), such that the long rep protein may take over some of the short rep protein function(s).

In some preferred embodiments, after transcription of the rep protein coding sequence, the ratio of long adeno-associated virus rep mRNA to short adeno-associated virus rep mRNA is about 1:15 to about 1:250, about 1:10 to about 1:225, about 1:10 to about 1:200, about 1:15 to about 1:200, about 1:20 to about 1:200, about 1:20 to about 1:150, about 1:25 to about 1:150, about 1:25 to about 1:125, about 1:25 to about 100, about 1:30 to about 1:95, about 1:35 to about 1:90, about 1:40 to about 1:85, about 1:45 to about 1:80, about 1:50 to about 1:75 or about 1:55 to about 1:70. In some embodiments, after transcription of the rep protein coding sequence, the ratio of long adeno-associated virus rep mRNA to short adeno-associated virus rep mRNA is about 1:15 to about 1:200, about 1:20 to about 1:200, about 1:20 to about 1:150, about 1:25 to about 1:150, about 1:25 to about 1:125, about 1:25 to about 100, about 1:30 to about 1:95, about 1:35 to about 1:90, about 1:40 to about 1:85, about 1:45 to about 1:80, about 1:50 to about 1:75 or about 1:55 to about 1:70. In some embodiments, after transcription of the rep protein coding sequence, the ratio of long adeno-associated virus rep protein mRNA to short adeno-associated virus rep mRNA is about 1:5 to about 1:100, about 1:5 to about 1:95, about 1:5 to about 1:90, about 1:5 to about 1:85, about 1:5 to about 1:80, about 1:5 to about 1:75, or about 1:5 to about 1:70. Optionally, in such embodiments, the long adeno-associated virus rep protein mRNA level is high.

In some embodiments, after transcription of the rep protein coding sequence, the ratio of long adeno-associated virus rep mRNA to short adeno-associated virus rep mRNA is about 1:300, about 1:275, about 1:250, about 1:200, about 1:175, about 1:150, about 1:125, about 1:100, about 1:90, about 1:80, about 1:70, about 1:60, about 1:50, about 1:45, about 1:40, about 1:35, about 1:30, about 1:25, about 1:20, about 1:15, about 1:10, about 1:9, about 1:8, about 1:7, about 1:6, or about 1:5. In some embodiments, after transcription of the rep protein coding sequence, the ratio of long adeno-associated virus rep mRNA to short adeno-associated virus rep mRNA is about 1:100, about 1:90, about 1:80, about 1:70, about 1:60, about 1:50, about 1:45, about 1:40, about 1:35, about 1:30, about 1:25, about 1:20, about 1:15, about 1:10, about 1:9, about 1:8, about 1:7, about 1:6, or about 1:5. Optionally, in such embodiments, the long adeno-associated virus rep protein mRNA level is high.

In some embodiments, after transcription of the rep protein coding sequence, the ratio of long adeno-associated virus rep protein mRNA to short adeno-associated virus rep protein mRNA is about 1:5 to about 1:5000. In some preferred embodiments, after transcription of the rep protein coding sequence, the ratio of long adeno-associated virus rep mRNA to short adeno-associated virus rep mRNA is about 1:20 to about 1:2500. In some more preferred embodiments, after transcription of the rep protein coding sequence, the ratio of long adeno-associated virus rep mRNA to short adeno-associated virus rep mRNA is about 1:50 to about 1:2000. In some most preferred embodiments, after transcription of the rep protein coding sequence, the ratio of long adeno-associated virus rep mRNA to short adeno-associated virus rep mRNA is about 1: 100 to about 1:1500. In such embodiment, the long adeno-associated virus rep protein mRNA level may be low. Specifically, it was found that for a low level of long adeno-associated virus rep protein mRNA, the ratio between long and short rep protein mRNA is about 1:5 or higher, preferably about 1:100 or higher. Such high ratios advantageously increase the potency.

In some preferred embodiments, after transcription of the rep protein coding sequence, the ratio of long adeno-associated virus rep mRNA to short adeno-associated virus rep mRNA is about 1:20 to about 1:4000, about 1:25 to about 1:3500, about 1:30 to about 1:3000, about 1:35 to about 1:2500, about 1:40 to about 1:2400, about 1:45 to about 2300, about 1:50 to about 1:2200, about 1:55 to about 1:2100, about 1:60 to about 1:2000, about 1:65 to about 1:1900, about 1:70 to about 1:1800 or about 1:75 to about 1:1700. In some embodiments, after transcription of the rep protein coding sequence, the ratio of long adeno-associated virus rep mRNA to short adeno-associated virus rep mRNA is about 1: 100 to about 1:1500. Optionally, in such embodiments, the long adeno-associated virus rep protein mRNA level is low.

In some embodiments, after translation, the ratio of long adeno-associated virus replication proteins to short adeno-associated virus replication proteins is
(i) about 1:5 to about 1:300, preferably about 1:5 to about 1:250, more preferably about 1:5 to about 1:200, even more preferably about 1:5 to about 1:150, and most preferably about 1:5 to about 1:100, optionally, wherein the long adeno-associated virus rep protein level is high; and/or
(ii) about 1:5 to about 1:5000, preferably about 1:20 to about 1:2500, more preferably about 1:50 to about 1:2000, and most preferably about 1: 100 to about 1: 1500, optionally, wherein the long adeno-associated virus rep protein level is low.

In some embodiments, after translation, the ratio of long adeno-associated virus replication proteins to short adeno-associated virus replication proteins is
(i) about 1:5 to about 1:300, preferably about 1:5 to about 1:250, more preferably about 1:5 to about 1:200, even more preferably about 1:5 to about 1:150, and most preferably about 1:5 to about 1:100, wherein the long adeno-associated virus rep protein level is high; or
(ii) about 1:5 to about 1:5000, preferably about 1:20 to about 1:2500, more preferably about 1:50 to about 1:2000, and most preferably about 1: 100 to about 1: 1500, wherein the long adeno-associated virus rep protein level is low.

In some embodiments, after translation, the ratio of long adeno-associated virus rep proteins to short adeno-associated virus rep proteins is about 1:5 to about 1:300. In some preferred embodiments, after translation, the ratio of long adeno-associated virus rep proteins to short adeno-associated virus rep proteins is about 1:5 to about 1:250. In some more preferred embodiments, after translation, the ratio of long adeno-associated virus rep proteins to short adeno-associated virus rep proteins is about 1:5 to about 1:200. In some even more preferred embodiments, after translation, the ratio of long adeno-associated virus rep proteins to short adeno-associated virus rep proteins is about 1:5 to about 1:150. In some most preferred embodiments, after translation, the ratio of long adeno-associated virus rep proteins to short adeno-associated virus rep proteins is about 1:5 to about 1:100. In such embodiment, the long adeno-associated virus rep protein level may be high. Specifically, it was found that for a high level of long adeno-associated virus rep protein, the ratio between long and short rep proteins is about 1:5 or higher. Without being bound to theory, it is hypothesized that the long rep protein has the same domains as the short rep protein in addition to DNA binding domain(s), such that the long rep protein may take over some of the short rep protein function(s).

In some embodiments, after translation, the ratio of long adeno-associated virus rep proteins to short adeno-associated virus rep proteins is about 1:15 to about 1:250, about 1:10 to about 1:225, about 1:10 to about 1:200, about 1:15 to about 1:200, about 1:20 to about 1:200, about 1:20 to about 1:150, about 1:25 to about 1:150, about 1:25 to about 1:125, about 1:25 to about 100, about 1:30 to about 1:95, about 1:35 to about 1:90, about 1:40 to about 1:85, about 1:45 to about 1:80, about 1:50 to about 1:75 or about 1:55 to about 1:70. In some embodiments, after translation, the ratio of long adeno-associated virus rep proteins to short adeno-associated virus rep proteins is about 1:15 to about 1:200, about 1:20 to about 1:200, about 1:20 to about 1:150, about 1:25 to about 1:150, about 1:25 to about 1:125, about 1:25 to about 100, about 1:30 to about 1:95, about 1:35 to about 1:90, about 1:40 to about 1:85, about 1:45 to about 1:80, about 1:50 to about 1:75 or about 1:55 to about 1:70. In some embodiments, after translation, the ratio of long adeno-associated virus rep proteins to short adeno-associated virus rep proteins is about 1:5 to about 1:100, about 1:5 to about 1:95, about 1:5 to about 1:90, about 1:5 to about 1:85, about 1:5 to about 1:80, about 1:5 to about 1:75, or about 1:5 to about 1:70. Optionally, in such embodiment the long adeno-associated virus rep protein level is high.

In some embodiments, after translation, the ratio of long adeno-associated virus rep proteins to short adeno-associated virus rep proteins is about 1:300, about 1:275, about 1:250, about 1:200, about 1:175, about 1:150, about 1:125, about 1:100, about 1:90, about 1:80, about 1:70, about 1:60, about 1:50, about 1:45, about 1:40, about 1:35, about 1:30, about 1:25, about 1:20, about 1:15, about 1:10, about 1:9, about 1:8, about 1:7, about 1:6, or about 1:5. In some embodiments, after translation, the ratio of long adeno-associated virus rep proteins to short adeno-associated virus rep proteins is about 1:100, about 1:90, about 1:80, about 1:70, about 1:60, about 1:50, about 1:45, about 1:40, about 1:35, about 1:30, about 1:25, about 1:20, about 1:15, about 1:10, about 1:9, about 1:8, about 1:7, about 1:6, or about 1:5. Optionally, in such embodiments, the long adeno-associated virus rep protein level is high.

In some embodiments, after translation, the ratio of long adeno-associated virus rep proteins to short adeno-associated virus rep proteins is about 1:5 to about 1:5000. In some preferred embodiments, after translation, the ratio of long adeno-associated virus rep proteins to short adeno-associated virus rep proteins is about 1:20 to about 1:2500. In some more preferred embodiments, after translation, the ratio of long adeno-associated virus rep proteins to short adeno-associated virus rep proteins is about 1:50 to about 1:2000. In some most preferred embodiments, after translation, the ratio of long adeno-associated virus rep proteins to short adeno-associated virus rep proteins is about 1: 100 to about 1: 1500. In such embodiments, the long adeno-associated virus rep protein level may be low. Specifically, it was found that for a low level of long adeno-associated virus rep protein, the ratio between long and short rep proteins is about 1:5 or higher, preferably about 1:100 or higher. Such high ratios advantageously increase the potency.

In some embodiments, after translation, the ratio of long adeno-associated virus rep proteins to short adeno-associated virus rep proteins is about 1:20 to about 1:4000, about 1:25 to about 1:3500, about 1:30 to about 1:3000, about 1:35 to about 1:2500, about 1:40 to about 1:2400, about 1:45 to about 2300, about 1:50 to about 1:2200, about 1:55 to about 1:2100, about 1:60 to about 1:2000, about 1:65 to about 1:1900, about 1:70 to about 1:1800 or about 1:75 to about 1: 1700. In some preferred embodiments, after translation, the ratio of long adeno-associated virus rep proteins to short adeno-associated virus rep proteins is about 1: 100 to about 1:1500. Optionally, in such embodiment the long adeno-associated virus rep protein level is low.

In some embodiments, after translation, the ratio of long adeno-associated virus rep proteins to short adeno-associated virus rep proteins is about 1: 100 or higher, about 1:200 or higher, about 1:300 or higher, about 1:400 or higher, about 1:500 or higher, about 1:600 or higher, about 1:700 or higher, about 1:800 or higher, about 1:900 or higher, or about 1: 1000 or higher. Optionally, in such embodiment the long adeno-associated virus rep protein level is low.

The rep plasmid of the invention can comprise at least one long adeno-associated virus rep protein coding sequence encoding at least one long functional rep protein and at least one short adeno-associated virus rep protein coding sequence encoding at least one short functional rep protein. In some embodiments, the long adeno-associated virus rep protein coding sequence encodes at least two long functional rep proteins. In some embodiments, the short adeno-associated virus rep protein coding sequence encodes at least two short functional rep proteins. In some embodiments, the rep plasmid comprises at least two long adeno-associated virus replication protein coding sequences, each encoding at least one long functional rep protein. In some embodiments, the rep plasmid comprises at least two short adeno-associated virus replication protein coding sequences, each encoding at least one short functional rep protein.

In some embodiments, the long adeno-associated virus rep protein coding sequence encodes at least one long functional rep protein selected from rep68 and rep78 or artificial variants thereof. In some preferred embodiments, the long functional rep protein is selected from rep68 or an artificial variant thereof.

In some embodiments, the short adeno-associated virus rep protein coding sequence encodes at least one short functional rep protein selected from rep40 and rep52 or artificial variants thereof. In some preferred embodiments, the short functional rep protein is selected from rep52 or an artificial variant thereof.

For example, the rep plasmid comprises a long adeno-associated virus rep protein coding sequence encoding the functional rep protein selected from rep68 and rep78 or artificial variants thereof, and a short adeno-associated virus rep protein coding sequence encoding the short functional rep protein selected from rep40 and rep52 or artificial variants thereof.

In some embodiments, the rep plasmid comprises a long adeno-associated virus rep protein coding sequence encoding the functional rep proteins rep68 and rep78 or artificial variants thereof, and a short adeno-associated virus rep protein coding sequence encoding the short functional rep proteins rep40 and rep52 or artificial variants thereof. In some embodiments, the rep plasmid comprises a long adeno-associated virus rep protein coding sequence encoding the functional rep protein rep68 or an artificial variant thereof, and a short adeno-associated virus rep protein coding sequence encoding the short functional rep proteins rep40 and rep52 or artificial variants thereof. In some embodiments, the rep plasmid comprises a long adeno-associated virus rep protein coding sequence encoding the functional rep protein rep78 or an artificial variant thereof, and a short adeno-associated virus rep protein coding sequence encoding the short functional rep proteins rep40 and rep52 or artificial variants thereof. In some embodiments, the rep plasmid comprises a long adeno-associated virus rep protein coding sequence encoding the functional rep proteins rep68 and rep78 or artificial variants thereof, and a short adeno-associated virus rep protein coding sequence encoding the short functional rep protein rep40 or an artificial variant thereof. In some embodiments, the rep plasmid comprises a long adeno-associated virus rep protein coding sequence encoding the functional rep proteins rep68 and rep78 or artificial variants thereof, and a short adeno-associated virus rep protein coding sequence encoding the short functional rep protein rep52 or an artificial variant thereof.

In some embodiments, the rep plasmid comprises a long adeno-associated virus rep protein coding sequence encoding a functional rep68 protein or an artificial variant thereof, and a short adeno-associated virus rep protein coding sequence encoding a functional rep40 protein or an artificial variant thereof. In some preferred embodiments, the rep plasmid comprises a long adeno-associated virus rep protein coding sequence encoding a functional rep68 protein or an artificial variant thereof, and a short adeno-associated virus rep protein coding sequence encoding a functional rep52 protein or an artificial variant thereof. In some embodiments, the rep plasmid comprises a long adeno-associated virus rep protein coding sequence encoding a functional rep78 protein or an artificial variant thereof, and a short adeno-associated virus rep protein coding sequence encoding a functional rep40 protein or an artificial variant thereof. In some embodiments, the rep plasmid comprises a long adeno-associated virus rep protein coding sequence encoding a functional rep78 protein or an artificial variant thereof, and a short adeno-associated virus rep protein coding sequence encoding a functional rep52 protein or an artificial variant thereof.

In some preferred embodiments, the rep plasmid does not comprise a long adeno-associated virus rep protein coding sequence encoding a functional rep78 protein or an artificial variant thereof. In some preferred embodiments, the rep plasmid does not comprise a short adeno-associated virus rep protein coding sequence encoding a functional rep40 protein or an artificial variant thereof.

In some embodiments, the rep plasmid comprises:
a) a single long adeno-associated virus rep protein coding sequence encoding a functional rep78 protein or rep68 protein or artificial variants thereof, and
b) a single short adeno-associated virus rep protein coding sequence encoding a functional rep52 protein or rep40 protein or artificial variants thereof.

In some preferred embodiments, the rep plasmid comprises:
a) a single long adeno-associated virus rep protein coding sequence encoding a functional rep68 protein or an artificial variant thereof, and
b) a single short adeno-associated virus rep protein coding sequence encoding a functional rep52 protein or an artificial variant thereof.

In some preferred embodiments, the rep plasmid does not comprise:
a) a long adeno-associated virus rep protein coding sequence encoding a functional rep78 protein or an artificial variant thereof, and
b) a short adeno-associated virus rep protein coding sequence encoding a functional rep40 protein or an artificial variant thereof.

In some embodiments, the long and/or short rep protein coding sequence is derived from an AAV serotype selected from the group consisting of AAV type 1 (e.g., AAV of serotype 1, also referred to as AAV1), AAV type2 (e.g., AAV2), AAV type 3 (e.g., AAV3, including types 3A and 3B, AAV3A and AAV3B), AAV type 4 (e.g., AAV4), AAV type 5 (e.g., AAV5), AAV type 6 (e.g., AAV 6), AAV type 7 (e.g., AAV7), AAV type 8 (e.g., AAV8), AAV type 9 (e.g., AAV9), AAV type 10 (e.g., AAV10), AAV type 11 (e.g., AAV 11), AAV type 12 (e.g., AAV 12), AAV type 13 (e.g., AAV 13), AAV type rh32.33 (e.g., AAVrh32.33), AAV type rh8 (e.g., AAVrh8), AAV type rhlO (e.g., AAVrhlO), AAV type rh74 (e.g., AAVrh74), AAV type hu.68 (e.g., AAVhu.68), avian AAV (e.g., AAAV), bovine AAV (e.g., BAAV), canine AAV, equine AAV, ovine AAV, snake AAV, bearded dragon AAV, AAV2i8, AAV2g9, AAV-LK03, AAV7m8, AAV Anc80, AAV PHP.B, and any other AAV now known or later discovered. In some preferred embodiments, the long and short adeno-associated virus rep protein coding sequences are derived from an AAV serotype selected from the group consisting of AAV-1, AAV-2, AAV-3, AAV-4, AAV-5, AAV-6, AAV-7 and AAV-8. In some preferred embodiments, the long or short adeno-associated virus rep protein coding sequences are derived from an AAV serotype selected from the group consisting of AAV-1, AAV-2, AAV-3, AAV-4, AAV-5, AAV-6, AAV-7, and AAV-8. In some more preferred embodiments, the long and short adeno-associated virus rep protein coding sequences are derived from an AAV2 serotype. In some more preferred embodiments, the long or short adeno-associated virus rep protein coding sequences are derived from an AAV2 serotype.

In some embodiments, the long and short rep protein sequence comprises or consists of a sequence shown in any of SEQ ID NO: 1-4 or a sequence having at least 60%, at least 70%, at least 80%, or at least 90% identity to said SEQ ID NOs. In some embodiments, the rep78 protein comprises or consists of the SEQ ID NO: 1 or a sequence having at least 60%, at least 70%, at least 80%, or at least 90% identity to said SEQ ID NO. In some embodiments, the rep68 protein comprises or consists of the SEQ ID NO: 2 or a sequence having at least 60%, at least 70%, at least 80%, or at least 90% identity to said SEQ ID NO. In some embodiments, the rep52 protein comprises or consists of the SEQ ID NO: 3 or a sequence having at least 60%, at least 70%, at least 80%, or at least 90% identity to said SEQ ID NO. In some embodiments, the rep40 protein comprises or consists of the SEQ ID NO: 4 or a sequence having at least 60%, at least 70%, at least 80%, or at least 90% identity to said SEQ ID NO.

In some embodiments, the rep plasmid comprises a 5'UTR upstream of the long adeno-associated virus rep protein coding sequence. The rep plasmid can e.g., comprise a 5'UTR immediately upstream of the long adeno-associated virus rep protein coding sequence. In the context of this disclosure, immediately upstream can mean that the 5'UTR can be located at the 5' end of the long adeno-associated virus rep protein coding sequence. In some preferred embodiments, the 5'UTR is derived from the sequence located between the native p5 promoter and the coding sequence of the wildtype long adeno-associated virus rep protein.

In some embodiments, the 5' UTR of the long adeno-associated virus rep protein coding sequence comprises a rep-binding element (RBE) and/or a terminal resolution site (TRS) derived from the native p5 promoter.

In some embodiments, the rep plasmid comprises a 5'UTR upstream of the short adeno-associated virus rep protein coding sequence. The rep plasmid can e.g., comprise a 5'UTR immediately upstream of the short adeno-associated virus rep protein coding sequence. In the context of this disclosure, immediately upstream can mean that the 5'UTR can be located at the 5' end of the short adeno-associated virus rep protein coding sequence. In some preferred embodiments, the 5'UTR is derived from the sequence located between the native p19 promoter and the coding sequence of the wildtype short adeno-associated virus rep protein.

In some embodiments, the rep plasmid comprises a 3'UTR downstream of the long and/or short adeno-associated virus rep protein coding sequence. The rep plasmid can e.g., comprise a 3'UTR immediately downstream of the short adeno-associated virus rep protein coding sequence. For example, the rep plasmid can comprise a 3'UTR immediately downstream of the long adeno-associated virus rep protein coding sequence. In some embodiments, the rep plasmid can comprise a 3'UTR immediately downstream of the long and short adeno-associated virus rep protein coding sequence. In the context of this disclosure, immediately downstream can mean that the 3'UTR can be located at the 3' end of the long and/or short adeno-associated virus rep protein coding sequence. In some preferred embodiments, the 3'UTR of the long and short adeno-associated virus rep protein coding sequence is derived from the sequence located between the wildtype capsid protein coding sequence and the wildtype 3'ITR. In some preferred embodiments, the 3'UTR of the long or short adeno-associated virus rep protein coding sequence is derived from the sequence located between the wildtype capsid protein coding sequence and the wildtype 3'ITR. In some embodiments, the wildtype capsid protein coding sequence is derived from the AAV2 genome. In some embodiments, the wildtype 3'ITR is derived from the AAV2 genome. In some embodiments, the wildtype 3'ITR comprises a sequence set forth in SEQ ID NO: 11 or a sequence having at least 60%, at least 70%, at least 80%, or at least 90% identity to said SEQ ID NO.

In some embodiments, the 3' UTR of the long and/or short adeno-associated virus rep protein coding sequence comprises one or more polyadenylation signal sequences, one or more RBEs, and/or one or more p5 promoters. In some embodiments, the 3' UTR of the long and short adeno-associated virus rep protein coding sequence comprises one or more polyadenylation signal sequences, one or more RBEs, and/or one or more p5 promoters. In some embodiments, the 3' UTR of the long or short adeno-associated virus rep protein coding sequence comprises one or more polyadenylation signal sequences, one or more RBEs, and/or one or more p5 promoters.

In some embodiments, the 3'UTR of the long and/or short adeno-associated virus rep protein coding sequence comprises one or more polyadenylation signal sequences. In some embodiments, the one or more polyadenylation signal sequence is selected from a native and/or a heterologous polyadenylation signal sequence. In some embodiments, the one or more polyadenylation signal sequence is selected from a native and a heterologous polyadenylation signal sequence. In some embodiments, the one or more polyadenylation signal sequence is selected from a native or a heterologous polyadenylation signal sequence.

In some embodiments the 3' UTR of the long and/or short adeno-associated virus rep protein coding sequence comprises a heterologous element, preferably a heterologous polyadenylation signal sequence. In some preferred embodiments, the heterologous polyadenylation signal sequence is a strong polyadenylation signal sequence. In some preferred embodiments, the 3' UTR of the long and/or short adeno-associated virus rep protein coding sequence comprises a strong heterologous polyadenylation signal sequence, e.g., SV40 or rabbit β-globin poly(A) signal sequence. In some preferred embodiments, the strong heterologous polyadenylation signal sequence comprised in the 3' UTR of the long and/or short adeno-associated virus rep protein coding sequence comprises or consists of a sequence set forth in SEQ ID NO: 23 or a sequence having at least 60%, at least 70%, at least 80%, or at least 90% identity to said SEQ ID NO. In some preferred embodiments, the strong heterologous polyadenylation signal sequence comprised in the 3' UTR of the long and short adeno-associated virus rep protein coding sequence comprises or consists of a sequence set forth in SEQ ID NO: 23 or a sequence having at least 60%, at least 70%, at least 80%, or at least 90% identity to said SEQ ID NO. In some preferred embodiments, the strong heterologous polyadenylation signal sequence comprised in the 3' UTR of the long or short adeno-associated virus rep protein coding sequence comprises or consists of a sequence set forth in SEQ ID NO: 23 or a sequence having at least 60%, at least 70%, at least 80%, or at least 90% identity to said SEQ ID NO.

In some embodiments, the 3' UTR of the long and/or short adeno-associated virus rep protein coding sequence comprises one or more polyadenylation signal sequences. In some embodiments, the 3' UTR of the long and/or short adeno-associated virus rep protein coding sequence comprises at least two polyadenylation signal sequences. In some embodiments, the 3' UTR of the long and/or short adeno-associated virus rep protein coding sequence comprises two polyadenylation signal sequences. In such embodiments, the (at least) two polyadenylation signal sequences can either be the same polyadenylation signal sequence or can be different polyadenylation signal sequences. For example, the 3' UTR of the long and/or short adeno-associated virus rep protein coding sequence can comprise a first native polyadenylation signal sequence and a second strong heterologous polyadenylation signal sequence, e.g., a rabbit β-globin poly(A) signal sequences as shown in SEQ ID NO: 23. Also, the 3' UTR of the long and short adeno-associated virus rep protein coding sequence can comprise a first native polyadenylation signal sequence and a second strong heterologous polyadenylation signal sequence, e.g., a rabbit β-globin poly(A) signal sequences as shown in SEQ ID NO: 23. Furthermore, the 3' UTR of the long or short adeno-associated virus rep protein coding sequence can comprise a first native polyadenylation signal sequence and a second strong heterologous polyadenylation signal sequence, e.g., a rabbit β-globin poly(A) signal sequences as shown in SEQ ID NO: 23.

In some embodiments, the 3' UTR of the long and/or short adeno-associated virus rep protein coding sequence comprises one or more RBEs, preferably the one or more RBE is derived from the p5 promoter.

In some embodiments, the one or more RBE comprised in the 3' UTR of the long and/or short adeno-associated virus rep protein coding sequence consists of the sequence shown in SEQ ID NO: 12. In some embodiments, the one or more RBE comprised in the 3' UTR of the long and short adeno-associated virus rep protein coding sequence consists of the sequence shown in SEQ ID NO: 12. In some embodiments, the one or more RBE comprised in the 3' UTR of the long or short adeno-associated virus rep protein coding sequence consists of the sequence shown in SEQ ID NO: 12. In some embodiments, the 3' UTR of the long and/or short adeno-associated virus rep protein coding sequence comprises an RBE derived from the p5 promoter and consisting of the sequence shown in SEQ ID NO: 12. In some embodiments, the 3' UTR of the long and short adeno-associated virus rep protein coding sequence comprises an RBE derived from the p5 promoter and consisting of the sequence shown in SEQ ID NO: 12. In some embodiments, the 3' UTR of the long or short adeno-associated virus rep protein coding sequence comprises an RBE derived from the p5 promoter and consisting of the sequence shown in SEQ ID NO: 12.

In some embodiments, the 3' UTR of the long and/or short adeno-associated virus rep protein coding sequence comprises one or more p5 promoters or variants thereof.

In some embodiments, the one or more p5 promoters or variants thereof comprised in the 3' UTR of the long and/or short adeno-associated virus rep protein coding sequence comprises or consists of a sequence set forth in SEQ ID NO: 17 or a sequence having at least 60%, at least 70%, at least 80%, or at least 90% identity to said SEQ ID NO. In some embodiments, the one or more p5 promoters or variants thereof comprised in the 3' UTR of the long and short adeno-associated virus rep protein coding sequence comprises or consists of a sequence set forth in SEQ ID NO: 17 or a sequence having at least 60%, at least 70%, at least 80%, or at least 90% identity to said SEQ ID NO. In some embodiments, the one or more p5 promoters or variants thereof comprised in the 3' UTR of the long or short adeno-associated virus rep protein coding sequence comprises or consists of a sequence set forth in SEQ ID NO: 17 or a sequence having at least 60%, at least 70%, at least 80%, or at least 90% identity to said SEQ ID NO.

In some embodiments, the 3' UTR of the long and/or short adeno-associated virus rep protein coding sequence comprises a p5 promoter or a variant thereof. In some embodiments, the p5 promoter or variant thereof comprised in the 3' UTR of the long and/or short adeno-associated virus rep protein coding sequence comprises or consists of a sequence set forth in SEQ ID NO: 17 or a sequence having at least 60%, at least 70%, at least 80%, or at least 90% identity to said SEQ ID NO. In some embodiments, the p5 promoter or variant thereof comprised in the 3' UTR of the long and short adeno-associated virus rep protein coding sequence comprises or consists of a sequence set forth in SEQ ID NO: 17 or a sequence having at least 60%, at least 70%, at least 80%, or at least 90% identity to said SEQ ID NO. In some embodiments, the p5 promoter or variant thereof comprised in the 3' UTR of the long or short adeno-associated virus rep protein coding sequence comprises or consists of a sequence set forth in SEQ ID NO: 17 or a sequence having at least 60%, at least 70%, at least 80%, or at least 90% identity to said SEQ ID NO.

The rep plasmid of the invention can comprise one or more polyadenylation signal sequences. In some embodiments, the rep plasmid comprises a first heterologous polyadenylation signal sequence as the heterologous element and one or more additional polyadenylation signal sequences.

In some embodiments, the polyadenylation signal sequence can be a sequence comprising AATAAA or a modified sequence thereof. The modified sequence of AATAAA can be a sequence in which one or two nucleic acids are deleted, substituted, inserted and/or added. Other polyadenylation signal sequences are known, for example, ATTAAA, AGTAAA, TATAAA, CATAAA, GATAAA, AATATA, AATACA, AATAGA, AAAAAG, and ACTAAA, and can be used in context of the present disclosure.

In some embodiments, the one or more additional polyadenylation signal sequence is capable of forming a proper poly(A) sequence at the RNA's 3' end. Examples of polyadenylation signal sequences are, without being limited, an adenovirus L3 poly(A) signal sequence, HSV TK poly(A) signal sequence, hGH poly(A) signal sequence, spA poly(A) signal sequence, rabbit gbpA poly(A) signal sequence, sNRP1 poly(A) signal sequence, bGH poly(A) signal sequence, synthetic poly(A) signal sequence, mouse β-globin poly(A) signal sequence, rabbit β-globin poly(A) signal sequence, H4-based poly(A) signal sequence, and SV40 poly(A) signal sequence. In some embodiments, the polyadenylation signal sequence is a SV40 poly(A) signal sequence.

In some embodiments, the rep plasmid comprises at least one adeno-associated virus cap protein coding sequence encoding at least one functional cap protein. In some embodiments, the adeno-associated virus cap protein coding sequence encodes at least one functional cap protein selected from VP1, VP2, and VP3, or artificial variants thereof or any combination thereof.

In some embodiments, the adeno-associated virus cap protein coding sequence is derived from an AAV serotype selected from the group consisting of AAV type 1 (e.g., AAV of serotype 1, also referred to as AAV1), AAV type2 (e.g., AAV2), AAV type 3 (e.g., AAV3, including types 3A and 3B, AAV3A and AAV3B), AAV type 4 (e.g., AAV4), AAV type 5 (e.g., AAV5), AAV type 6 (e.g., AAV 6), AAV type 7 (e.g., AAV7), AAV type 8 (e.g., AAV8), AAV type 9 (e.g., AAV9), AAV type 10 (e.g., AAV10), AAV type 11 (e.g., AAV 11), AAV type 12 (e.g., AAV 12), AAV type 13 (e.g., AAV 13), AAV type rh32.33 (e.g., AAVrh32.33), AAV type rh8 (e.g., AAVrh8), AAV type rhlO (e.g., AAVrhlO), AAV type rh74 (e.g., AAVrh74), AAV type hu.68 (e.g., AAVhu.68), avian AAV (e.g., AAAV), bovine AAV (e.g., BAAV), canine AAV, equine AAV, ovine AAV, snake AAV, bearded dragon AAV, AAV2i8, AAV2g9, AAV-LK03, AAV7m8, AAV Anc80, AAV PHP.B, and any other AAV now known or later discovered. In some preferred embodiments, the adeno-associated virus cap protein coding sequence is derived from an AAV serotype selected from the group consisting of AAV-1, AAV-2, AAV-3, AAV-4, AAV-5, AAV-6, AAV-7, and AAV-8. In some more preferred embodiments, the adeno-associated virus cap protein coding sequence is derived from an AAV2 serotype.

In some embodiments, the cap protein sequence comprises or consists of a sequence shown in any of SEQ ID NO: 5-7 or a sequence having at least 60%, at least 70%, at least 80%, or at least 90% identity to said SEQ ID NOs. In some embodiments, the VP1 protein comprises or consists of the SEQ ID NO: 5 or a sequence having at least 60%, at least 70%, at least 80%, or at least 90% identity to said SEQ ID NO. In some embodiments, the VP2 protein comprises or consists of the SEQ ID NO: 6 or a sequence having at least 60%, at least 70%, at least 80%, or at least 90% identity to said SEQ ID NO. In some embodiments, the VP3 protein comprises or consists of the SEQ ID NO: 7 or a sequence having at least 60%, at least 70%, at least 80%, or at least 90% identity to said SEQ ID NO.

In some embodiments, the rep plasmid comprises at least one accessory protein coding sequence encoding at least one functional accessory protein.

The accessory protein can be selected from AAP (Assembly-Activating Protein), MAAP (Membrane-Associated Accessory Protein), and X or variants thereof or any combination thereof. The coding sequences of the accessory protein overlap with the capsid gene in the wild-type AAV. AAP is suggested to be important for assembly of the capsid and maintaining capsid protein stability (Grosse, Stefanie et al. "Relevance of Assembly-Activating Protein for Adeno-associated Virus Vector Production and Capsid Protein Stability in Mammalian and Insect Cells." Journal of virology vol. 91,20 e01198-17. 27 Sep. 2017, doi:10.1128/JVI.01198-17). MAAP is commonly translated from a non-canonical start codon, CTG, and is suggested to be associated with the cell membrane. MAAP is contemplated to limit the production of other AAV strains through competitive exclusion (Karlin, D.G. Sequence Properties of the MAAP Protein and of the VP1 Capsid Protein of Adeno-Associated Viruses. Preprints 2020, 2020020234). The function of X is unknown, although X is suggested to be involved in DNA replication (Cao M, You H, Hermonat PL (2014) The X Gene of Adeno-Associated Virus 2 (AAV2) Is Involved in Viral DNA Replication. PLoS ONE 9(8): e104596).

In some embodiments, the accessory protein coding sequence encodes at least one functional accessory protein selected from AAP, MAAP, and X, or artificial variants thereof.

In some preferred embodiments, the accessory protein coding sequence encodes MAAP or an artificial variant thereof. In some embodiments, the accessory protein coding sequence encodes X or an artificial variant thereof. In some embodiments, the accessory protein coding sequence encodes AAP or an artificial variant thereof. The rep plasmid disclosed herein can comprise any combination of accessory protein coding sequences. For example, the rep plasmid can comprise a first accessory protein coding sequence encoding MAAP or an artificial variant thereof, and a second accessory protein coding sequence encoding X or an artificial variant thereof. The rep plasmid can comprise a first accessory protein coding sequence encoding AAP or an artificial variant thereof, and a second accessory protein coding sequence encoding X or an artificial variant thereof. The rep plasmid can comprise a first accessory protein coding sequence encoding MAAP or an artificial variant thereof, and a second accessory protein coding sequence encoding AAP or an artificial variant thereof. The rep plasmid can comprise a first accessory protein coding sequence encoding MAAP or an artificial variant thereof, a second accessory protein coding sequence encoding X or an artificial variant thereof, and a third accessory protein coding sequence encoding AAP or an artificial variant thereof. Preferably, the rep plasmid comprises at least one accessory protein coding sequence encoding MAAP or an artificial variant thereof.

In some embodiments, the MAAP protein comprises or consists of the SEQ ID NO: 9 or a sequence having at least 60%, at least 70%, at least 80%, or at least 90% identity to said SEQ ID NO. In some embodiments, the AAP protein comprises or consists of the SEQ ID NO: 8 or a sequence having at least 60%, at least 70%, at least 80%, or at least 90% identity to said SEQ ID NO. In some embodiments, the X protein comprises or consists of the SEQ ID NO: 10 or a sequence having at least 60%, at least 70%, at least 80%, or at least 90% identity to said SEQ ID NO.

The rep plasmid disclosed herein can comprise the structure shown in Fig. 9. The schematic representation of the rep plasmid depicted in Fig. 9 represents an exemplary structure of the rep plasmid and should not be construed to limit the scope of the rep plasmid of the invention.

### The rep-cap plasmid system of the invention

In another aspect, the invention further provides a rep-cap plasmid comprising:
(i) at least one long adeno-associated virus replication protein coding sequence encoding at least one long functional rep protein;
(ii) at least one short adeno-associated virus replication protein coding sequence encoding at least one short functional rep protein;
(iii) at least one adeno-associated virus capsid protein coding sequence encoding at least one functional cap protein; and
(iv) at least one heterologous element, wherein following transcription the at least one heterologous element results in a ratio of long adeno-associated virus rep mRNA to short adeno-associated virus rep mRNA of about 1:5 to about 1:5000.

The rep-cap plasmid disclosed herein can comprise the structure shown in Fig. 9. The schematic representation of the rep-cap plasmid depicted in Fig. 9 represents an exemplary structure of the rep-cap plasmid of the invention and should not be construed to limit the scope of the rep-cap plasmid of the invention.

The embodiments disclosed herein for the rep plasmid of the invention can be used for the rep-cap plasmid as disclosed herein. Features described herein in more detail in connection with the "rep plasmid" embodiments equally apply to the corresponding "rep-cap plasmid" embodiments.

### The plasmid system of the invention

In another aspect, the present invention provides a plasmid system for producing an adeno-associated virus particle comprising:
(i) the rep plasmid of the invention; and
(ii) a transgene plasmid.

In some embodiments, the plasmid system disclosed herein can be a two-plasmid system comprising all the necessary genetic information for the production of rAAV.

The two-plasmid system may comprise at least one long adeno-associated virus rep protein coding sequence encoding at least one long functional rep protein, at least one short adeno-associated virus rep protein coding sequence encoding at least one short functional rep protein, at least one adeno-associated virus cap protein coding sequence encoding at least one functional cap protein, at least one helper gene, and a transgene.

In some embodiments, the two-plasmid system can comprise:
(i) a rep plasmid of the invention, and
(ii) a transgene plasmid, and
wherein the rep plasmid further comprises at least one adeno-associated virus cap protein coding sequence encoding at least one functional cap protein, and wherein the transgene plasmid further comprises at least one helper gene.

In some embodiments, the two-plasmid system can comprise:
(i) a rep-cap plasmid of the invention, and
(ii) a transgene plasmid, and
wherein the transgene plasmid further comprises at least one helper gene.

In some embodiments, the two-plasmid system can comprise:
(i) a rep plasmid of the invention, and
(ii) a transgene plasmid, and
wherein the rep plasmid further comprises at least one adeno-associated virus cap protein coding sequence encoding at least one functional cap protein, and wherein the rep plasmid further comprises at least one helper gene.

In some embodiments, the two-plasmid system can comprise:
(i) a rep-cap plasmid of the invention, and
(ii) a transgene plasmid, and
wherein the rep-cap plasmid further comprises at least one helper gene.

In some embodiments, the transgene plasmid comprises a promoter, a transgene, a polyadenylation signal sequence, and 5' and 3' inverted terminal repeats,
wherein the transgene plasmid is selected from one of the following:
(i) conventional single-stranded genome recombinant adeno-associated virus, or
(ii) self-complementary genome recombinant adeno-associated adenovirus.

In some embodiments the transgene plasmid is a self-complementary AAV (scAAV) plasmid. Because the conventional ssAAV virus depends on the DNA replication machinery to synthesize the complementary DNA strand, transgene expression may be delayed. To overcome this rate-limiting step, scAAV contains complementary sequences that are capable of spontaneously annealing, upon infection, which eliminates the requirement for host cell DNA synthesis. Methods of generating scAAV plasmids are well known to the person skilled in the art. In some embodiments, the scAAV plasmid comprises a transgene of about 2.4 kb or less.

The transgene plasmid can comprise any suitable promoter. Exemplary suitable promoters can be a chicken β-actin (CBA) promoter, a short CMV early enhancer/chicken β actin (sCAG) promoter, human cytomegalovirus (hCMV) promoter, mouse phosphoglycerate kinase (mPGK) promoter, and human synapsin (hSYN) promoter. In some preferred embodiments, the promoter is a CMVie promoter.

In some embodiments, the transgene is a reporter gene. In some preferred embodiments, the reporter gene can be detected by antibody-based assays. In further preferred embodiments, the reporter gene is a fluorescent molecule. Exemplary fluorescent molecules suitable as reporter gene are GFP, eGFP, mGFP, eYFP, citrine, eCFP, mCFP, Cerulean, dtTomato, and any variants thereof. In some further preferred embodiments, the reporter gene is a beta-galactosidase, luciferase or glutathione S-transferase, or any variant thereof. In some preferred embodiments, the reporter gene comprises or consists of a sequence shown SEQ ID NO: 22 or preferably a sequence having at least 85% identity to said SEQ ID NO, preferably at least 90% identity to said SEQ ID NO, even more preferably at least 95% identity to said SEQ ID NO, most preferably at least 99% identity to said SEQ ID NO.

In some embodiments, the transgene has at most 4.7 kb. In some embodiments, the transgene has at most 2.4 kb.

The transgene plasmid can comprise any suitable polyadenylation signal sequence. In some embodiments, the polyadenylation signal sequence is capable of forming a proper poly(A) sequence at the RNA's 3' end. Exemplary polyadenylation signal sequences are an adenovirus L3 poly(A) signal sequence, HSV TK poly(A) signal sequence, hGH poly(A) signal sequence, spA poly(A) signal sequence, rabbit gbpA poly(A) signal sequence, sNRP1 poly(A) signal sequence, bGH poly(A) signal sequence, synthetic poly(A) signal sequence, mouse β-globin poly(A) signal sequence, rabbit β-globin poly(A) signal sequence, H4-based poly(A) signal sequence, and SV40 poly(A) signal sequence. In preferred embodiments, the polyadenylation signal sequence is a SV40 poly(A) signal sequence.

In some embodiments, the 3' and 5' inverted terminal repeats are independently derived from an ITR sequence of any AAV serotype, including but not limited to, AAV type 1 (e.g., AAV of serotype 1, also referred to as AAV1), AAV type2 (e.g., AAV2), AAV type 3 (e.g., AAV3, including types 3A and 3B, AAV3A and AAV3B), AAV type 4 (e.g., AAV4), AAV type 5 (e.g., AAV5), AAV type 6 (e.g., AAV 6), AAV type 7 (e.g., AAV7), AAV type 8 (e.g., AAV8), AAV type 9 (e.g., AAV9), AAV type 10 (e.g., AAV10), AAV type 11 (e.g., AAV 11), AAV type 12 (e.g., AAV 12), AAV type 13 (e.g., AAV 13), AAV type rh32.33 (e.g., AAVrh32.33), AAV type rh8 (e.g., AAVrh8), AAV type rhlO (e.g., AAVrhlO), AAV type rh74 (e.g., AAVrh74), AAV type hu.68 (e.g., AAVhu.68), avian AAV (e.g., AAAV), bovine AAV (e.g., BAAV), canine AAV, equine AAV, ovine AAV, snake AAV, bearded dragon AAV, AAV2i8, AAV2g9, AAV-LK03, AAV7m8, AAV Anc80, AAV PHP.B, and any other AAV now known or later discovered. In some preferred embodiments, the 3' and 5' inverted terminal repeats are independently derived from an ITR sequence of an AAV serotype selected from the group consisting of AAV-1, AAV-2, AAV-3, AAV-4, AAV-5, AAV-6, AAV-7 and AAV-8. In some preferred embodiments, the 3' and 5' inverted terminal repeats are derived from AAV2. In some more preferred embodiments, the 3' and 5' inverted terminal repeats are derived from J01901.1 AAV2.

In some embodiments, at least one inverted terminal repeat, preferably the 5' inverted terminal repeat, has a deletion compared to the native inverted terminal repeats. In some embodiments, the transgene plasmid comprising the 5'ITR deletion is a scAAV plasmid.

In some embodiments, the transgene plasmid comprises a promoter, a transgene, a polyadenylation signal sequence, 5' and 3' inverted terminal repeats and at least one helper gene.

In some embodiments, the plasmid system can comprise two or more transgene plasmids. The first transgene plasmid may comprise a 3' splice donor and the second a 5' splice acceptor. When both plasmids are expressed in a cell, they can form concatemers, are spliced together, and the full-length transgene can then be expressed. In other embodiments, a transgene is divided between two transgene plasmids, but with substantial sequence overlap. Co-expression can induce homologous recombination and expression of the full-length transgene.

In some embodiments, the rep plasmid disclosed herein or rep-cap plasmid disclosed herein comprises at least one helper gene. In some embodiments, the helper gene(s) do not comprise adenoviral late genes.

In another aspect, the plasmid system disclosed herein comprises a helper plasmid instead of the transgene plasmid, e.g., the plasmid system comprises at least two plasmids. In some embodiments the plasmid system can comprises:
(i) the rep plasmid of the invention; and
(ii) a helper plasmid, and
wherein the rep plasmid further comprises at least one adeno-associated virus cap protein coding sequence encoding at least one functional cap protein, and wherein the rep plasmid comprises at least one transgene.

In some aspects, the plasmid system can comprises:
(i) the rep-cap plasmid of the invention; and
(ii) a helper plasmid, and
wherein the rep-cap plasmid further comprises at least one transgene.

In some embodiments, the rep plasmid disclosed herein or rep-cap plasmid disclosed herein comprises at least one transgene.

Typically, AAV can be replication defective and may require co-infection by adenovirus or herpes virus in order to replicate efficiently. The individual adenoviral genes that contribute to AAV helper function are well-known and have been inter alia identified by testing adenovirus mutants for their ability to mediate AAV replication. In some embodiments, the helper plasmid comprises one or more coding sequences encoding for E1a, E1b, E2A, L4 22k, L4 33k, E4orf3, E4orf6, E4orf6-7 fusion, or virus-associated RNA or any combination thereof. These genes (i.e., helper genes) are known to support or participate in AAV replication. In some preferred embodiments, the helper plasmid comprises one or more coding sequences encoding for E2A, E4orf6, E4orf6-7 fusion, or virus-associated RNAs or any combination thereof. In some embodiments, the helper plasmid comprises one or more virus-associated RNAs. Adenovirus 'virus-associated' RNAs (VA RNAs) are suggested to be abundant, heterogeneous, non-coding RNA transcripts, typically comprising 150-200 nucleotides. For instance, VA RNAI is recognized for its function in relieving the cellular anti-viral blockade of protein synthesis through inhibition of the double-stranded RNA-activated protein kinase (PKR). More recent evidence has revealed that VA RNAs interfere with several other host cell processes.

In some embodiments, the helper plasmid does not comprise the adenoviral late genes. In the context of this disclosure, adenoviral late genes refer to adenovirus genes that are expressed at a later stage in the adenoviral reproduction cycle, i.e., after the expression of the early adenoviral genes, i.e., after the expression of the E1A, E1B, E2A, E2B, E3, and E4 genes. Without wishing to be bound by theory, it is contemplated that the adenoviral late genes encode for adenoviral capsid proteins and are involved in the assembly of adenoviral capsids. In some embodiments, the helper plasmid does not comprise the adenoviral L1-L5 late genes.

In some embodiments, the helper plasmid is not able to produce functional adenovirus particles, e.g., adenovirus serotype 5 particles.

In some embodiments, the plasmid system disclosed herein does not comprise an adenoviral vector or an adenoviral plasmid. In this context, an adenoviral vector or adenoviral plasmid refers to a vector or plasmid that is able to produce functional adenoviral particles, e.g., adenovirus serotype 5 particles. In some embodiments, the helper plasmid disclosed herein is not an adenoviral vector or an adenoviral plasmid.

In some embodiments, the helper genes can be selected from E1a, E1b, E2A, L4 22k, L4 33k, E4orf3, E4orf6, E4orf6-7 fusion, or virus-associated RNA or any combination thereof. In some preferred embodiments, the helper genes can be selected from E2A, E4orf6, E4orf6-7 fusion, or virus-associated RNAs or any combination thereof.

In some embodiments, the helper genes are not selected from adenoviral late genes. In some embodiments, the helper genes are not selected from adenoviral L1-L5 late genes. In some preferred embodiments, the rep plasmid disclosed herein does not comprise adenovirus late genes. In some preferred embodiments, the rep plasmid disclosed herein does not comprise adenoviral L1-L5 late genes. In some preferred embodiments, the transgene plasmid disclosed herein does not comprise adenovirus late genes. In some preferred embodiments, the transgene plasmid disclosed herein does not comprise adenoviral L1-L5 late genes.

In some embodiments, the helper genes are not able to produce functional adenovirus particles, e.g., adenovirus serotype 5 particles.

When using cells expressing the adenoviral E1A/B genes (such as HEK293T cells) the remaining adenoviral helper genes may encode E4, E2A and virus-associated RNA I and II.

In some embodiments, the helper plasmid comprises at least one transgene.

In some embodiments, the plasmid system comprises at least one accessory protein coding sequence encoding at least one functional accessory protein. In some embodiments, the rep plasmid or the rep-cap plasmid of the plasmid system comprises at least one accessory protein coding sequence encoding at least one functional accessory protein. In some embodiments, the rep plasmid or the rep-cap plasmid of the two-plasmid system comprises at least one accessory protein coding sequence encoding at least one functional accessory protein. In some embodiments, the accessory protein coding sequence encodes at least one functional accessory protein selected from AAP, MAAP, and X, or artificial variants thereof.

In some embodiments, the plasmid system comprises a helper plasmid, e.g., the plasmid system comprises at least three plasmids. In some embodiments the plasmid system comprises:
(i) the rep plasmid of the invention;
(ii) a transgene plasmid; and
(iii) a helper plasmid.

In some embodiments, the rep plasmid of the three-plasmid system further comprises at least one adeno-associated virus capsid protein coding sequence encoding at least one functional cap protein. In some embodiments, the adeno-associated virus capsid protein coding sequence encodes at least one functional cap protein selected from VP1, VP2, and VP3, or artificial variants thereof. In some preferred embodiments, the adeno-associated virus cap protein coding sequence is derived from an AAV serotype selected from the group consisting of AAV-1, AAV-2, AAV-3, AAV-4, AAV-5, AAV-6, AAV-7, and AAV-8. In some more preferred embodiments, the adeno-associated virus cap protein coding sequence is derived from an AAV2 serotype.

In some embodiments, the rep plasmid of the three-plasmid system further comprises at least one accessory protein coding sequence encoding at least one functional accessory protein. In some embodiments, the accessory protein coding sequence encodes at least one functional accessory protein selected from AAP, MAAP, and X, or artificial variants thereof.

In some embodiments, the three-plasmid system can comprise the rep-cap plasmid disclosed herein.

In some embodiments, the plasmid system comprises a cap plasmid, e.g., the plasmid system comprises at least four plasmids. In some embodiments the plasmid system comprises:
(i) the rep plasmid of the invention;
(ii) a transgene plasmid;
(iii) a helper plasmid; and
(iv) a cap plasmid.

In some embodiments, the cap plasmid of the four-plasmid system comprises at least one adeno-associated virus cap protein coding sequence encoding at least one functional cap protein. In some embodiments, the adeno-associated virus cap protein coding sequence encodes at least one functional cap protein selected from VP1, VP2, and VP3, or artificial variants thereof. In some preferred embodiments, the adeno-associated virus cap protein coding sequence is derived from an AAV serotype selected from the group consisting of AAV-1, AAV-2, AAV-3, AAV-4, AAV-5, AAV-6, AAV-7, and AAV-8. In some more preferred embodiments, the adeno-associated virus cap protein coding sequence is derived from an AAV2 serotype.

In some embodiments, the cap plasmid of the four-plasmid system comprises at least one accessory protein coding sequence encoding at least one functional accessory protein. In some embodiments, the rep plasmid comprises at least one accessory protein coding sequence encoding at least one functional accessory protein. In some embodiments, the accessory protein coding sequence encodes at least one functional accessory protein selected from AAP, MAAP, and X, or artificial variants thereof.

The embodiments disclosed herein for the rep plasmid and the rep-cap of the invention can be used for the plasmid system for producing an adeno-associated virus particle as disclosed herein. Features described herein in more detail in connection with the "rep plasmid" and the "rep-cap plasmid" embodiments equally apply to the corresponding "plasmid system" embodiments.

### The stable or transient cell expression system and the cell of the invention

In another aspect, the present invention provides a stable or transient cell expression system comprising the plasmid system of the invention and a cell line.

According to preferred embodiments, the stable expression system comprises the genetic elements of the plasmid system or the rep plasmid disclosed herein. For example, genome editing may be used to stably integrate one or more sequences present on the plasmid(s) or plasmid system disclosed herein into the chromosome of a cell to obtain a cell capable of stably producing an rAAV particle. The stable integration is advantageous in that it allows using the same stock of genome edited cells multiple times without the need to genetically modifying the cells before culturing the cells to produce rAAV particles. The integration can be performed by providing at least one site-directed endonuclease, preferably being selected from a meganuclease, a ZFN, a TALEN, a CRISPR-nuclease, a nickase, or nuclease-dead variant therefrom. The integration can be performed by providing at least one nucleic acid molecule encoding a site-directed endonuclease, preferably being selected from a meganuclease, a ZFN, a TALEN, a CRISPR-nuclease, a nickase, or nuclease-dead variant therefrom. CRISPR gene editing is well known to the skilled person. For example, CRISPR-directed gene integration can be conducted by providing at least one suitable, functional guide RNA molecule, or a nucleic acid molecule encoding the same, and genetic elements required for producing the rAAV in form of a template to be integrated into the genome. The template may be cleaved by the at least one site-directed endonuclease, e.g., a CRISPR-nuclease. The way of integrating the genetic elements into the genome of the host cell shall not be limited and different techniques are known to and available to the skilled person. According to particular embodiments, however, the one or more genetic elements required for viral vector production may be integrated using a CRISPR-nuclease, e.g., Cas12a or Cas9.

In some embodiments, the stable or transient cell expression system comprises the rep plasmid disclosed herein. In this context it is understood that the stable cell expression system does not comprise the plasmid as such but the respective gene(s) of the plasmid, e.g., sequences of a plasmid stably integrated into the genome of the cell. In some embodiments, the stable or transient cell expression system comprises the plasmid system disclosed herein.

In some embodiments, the stable or transient cell expression system comprises the rep plasmid disclosed herein and a transgene plasmid. In some embodiments, the stable or transient cell expression system comprises the rep-cap plasmid disclosed herein and a transgene plasmid.

In some embodiments, the stable or transient cell expression system comprises the rep plasmid disclosed herein and a helper plasmid. In some embodiments, the stable or transient cell expression system comprises the rep-cap plasmid disclosed herein and a helper plasmid.

In some embodiments, the stable or transient cell expression system comprises the rep plasmid disclosed herein and a cap plasmid. In some embodiments, the stable or transient cell expression system comprises the rep plasmid disclosed herein, a transgene plasmid, and a helper plasmid. In some embodiments, the stable or transient cell expression system comprises the rep-cap plasmid disclosed herein, a transgene plasmid, and a helper plasmid.

In some embodiments, the stable or transient cell expression system comprises the rep plasmid disclosed herein, a transgene plasmid, a helper plasmid, and a cap plasmid.

In some preferred embodiments, a cell expression system is provided wherein the sequence comprising the rep coding sequence is stably integrated. For example, the cell can provide a stable rep expression system and provide a transient cap, helper, and transgene expression system.

In another aspect, the present invention provides a cell comprising the rep plasmid of the invention, the plasmid system of the invention, or the rep-cap plasmid of the invention.

In some embodiments, the cell comprises the rep plasmid disclosed herein. In some embodiments, the cell comprises the plasmid system disclosed herein. In some embodiments, the cell comprises the rep-cap plasmid disclosed herein.

In some embodiments, the cell comprises the rep plasmid disclosed herein and a transgene plasmid. In some embodiments, the cell comprises the rep-cap plasmid disclosed herein and a transgene plasmid.

In some embodiments, the cell comprises the rep plasmid disclosed herein and a helper plasmid. In some embodiments, the cell comprises the rep-cap plasmid disclosed herein and a helper plasmid.

In some embodiments, the cell comprises the rep plasmid disclosed herein and a cap plasmid. In some embodiments, the cell comprises the rep plasmid disclosed herein, a transgene plasmid, and a helper plasmid. In some embodiments, the cell comprises the rep-cap plasmid disclosed herein, a transgene plasmid, and a helper plasmid.

In some embodiments, the cell comprises the rep plasmid disclosed herein, a transgene plasmid, a helper plasmid, and a cap plasmid.

In some preferred embodiments, the cell is a mammalian cell as described below. The cells can be HEK293 cells, HELA cells or insect cells, or derivatives thereof. Most preferably, the cell is a HEK293 cell.

The embodiments disclosed herein for the rep plasmid and the plasmid system of the invention can be used for the stable or transient cell expression system and the cell as disclosed herein. Features described herein in more detail in connection with the "rep plasmid" and the "plasmid system" embodiments equally apply to the corresponding "stable or transient cell expression system and cell" embodiments.

### The kit of the invention

In another aspect, the present invention provides a kit comprising a stable or transient cell expression system of the invention, or a cell of the invention, and a cell culture medium.

In some embodiments, the kit further comprises media feeds, media additives or transfection reagents or any combination thereof. In some embodiments, the kit further comprises a manual.

The embodiments disclosed herein for the stable or transient cell expression system or the cell of the invention can be used for the kit as disclosed herein. Features described herein in more detail in connection with the "stable or transient cell expression system" and the "cell" embodiments equally apply to the corresponding "kit" embodiments.

### The method of producing recombinant adeno-associated viral vectors of the invention

In another aspect, the present invention provides a method of producing recombinant adeno-associated viral vectors comprising:
(i) Transfecting cells with the rep plasmid of the invention, the plasmid system of the invention, or the rep-cap plasmid of the invention;
(ii) culturing the transfected cells to produce said adeno-associated viral vectors; and
(iii) isolating said recombinant adeno-associated viral vectors.

In some embodiments, the cells are HEK293 cells, HELA cells, CHO cells, insect cells, or derivatives thereof. For example, the insect cells can be SF9 cells.

In some embodiments, the cells are mammalian cells. A mammalian cell is a cell that is of mammalian origin. However, the cell does not need to be identical to a cell obtained in a mammalian but can be modified, engineered or naturally/artificially changed. For instance, the mammalian cell may be a cell with mammalian origin, however, may contain one or more genetic changes to propagate it repeatedly and possibly also infinitely. The term "mammalian cell" may be interchangeable used with "mammalian cells" and shall cover both the singular and plural form. The mammalian cell is preferably a mammalian cell line. According to a preferred embodiment, the mammalian cell is a human-derived cell line, which advantageously has the ability to provide the suitable post-translational modifications for therapy in humans. This means that the produced viral vector has similar post-translational modification, as if the native virus would have infected a human cell resulting in virus production.

The mammalian cell can be selected from the group consisting of HeLa, Human embryonic kidney 293 (HEK293), BSC-1, SW480, Baby hamster kidney (BHK), BHK-21, Vero E6, U2OS, A549, HT1080, CAD, P19, NIH 3T3, L929, N2a, Chinese hamster ovary (CHO), MCF-7, Y79, SO-Rb50, Hep G2, DUKX-X11, J558L, HuH-7, MDCK, or HepG2 cells or derivatives thereof. According to a preferred embodiment, the mammalian cell is selected from the group consisting of HEK293, A549, BSC-1, SW480, Baby hamster kidney (BHK), Vero E6 and MDCK cells or a derivative thereof.

While "HEK293" are established in the field for viral vector production, since their creation in the 1970ies various derivatives thereof have been generated, which are also well suited for viral vector production. In general, according to preferred embodiments, the derivative of the HEK293 cell is characterized by being a cell that roots back to HEK293 and/or was originally a HEK293 cell that was adapted, modified, differentiated, reprogrammed, transformed, transduced, transfected and/or mutated. The skilled person can readily identify a HEK293 cell as known in the art, as it typically contains the adenovirus 5 DNA inserted in human chromosome 19. While it is not excluded that such adenovirus 5 DNA insert has been modified, it is believed that certain similarities will always be existent in order to identify a HEK293 cell or a respective derivative thereof. According to preferred embodiments, the derivative of the HEK293 cell is selected from one or more of the group consisting of 293, 293T, 293T/17, ANJOU 65, 293H, 293E, 293-6E, EBNA1-6E, 293F, 293FT, 293Flp-IN T-REx, 293FTM, 293 S, 293SG, 293SGGD, 293MSR, 293A, or any modified variants thereof. Such derivatives may be advantageous for viral vector production. Some of these derivatives may have been particularly adapted in the past in order to enhance viral vector production.

Transfection reagents support the introduction of exogenous genetic material into a target cell, e.g., a eucaryotic cell. Exogenous genetic material is usually provided on a nucleic acid construct (e.g., plasmid, DNA linear or ligated double-stranded, messenger RNA). As eukaryotic cells are not permeable to nucleic acids, delivery systems or vectors allowing their introduction have been developed. Besides viral infection systems (e.g., infection by baculovirus) and physical methods (e.g., electroporation), chemical transfection reagents are commonly used to introduce exogenous genetic material into eukaryotic target cells.

In some embodiments, in step (i) of the method according to the invention for producing recombinant adeno-associated viral vectors, one or more transfection reagents are used to introduce, i.e., to transfect, the cells with the rep plasmid of the invention, the plasmid system of the invention, or the rep-cap plasmid of the invention. Without wishing to be bound by theory, the use of transfection reagents in the method for producing rAAV particles according to the invention is contemplated to improve the transfer of the rep plasmid of the invention, the plasmid system of the invention, or the rep-cap plasmid of the invention into the target cell, and thereby improve the viral genome titers (vg/L), viral capsid titers (cp/L), and transducing titers (TU/L) of the rAAV particles produced by the transfected cell.

In some embodiments, the one or more transfection reagent used in the method of the invention is selected from commercially available transfection reagents such as LipoFectAmine^{®} 2000 & 3000 (ThermoFisher), TransIT reagents^{®}(MirusBio), FuGene^{®} (Promega), XtremeGene^{®} (Roche), jetPRIME^{®} (Polyplus-transfection), ViaFect^{®} (Promega), FectoVIR^{®} (Polyplus-transfection), or PEIpro^{®} (Polyplus-transfection).

In some embodiments, the one or more transfection reagent used in the method of the invention is selected from any of the transfection reagents disclosed in WO 2021/023798 A1, which is hereby incorporated by reference in its entirety. In some embodiments, the one or more transfection reagent used in the method of the invention is selected from any of the transfection reagents disclosed in WO 2023/161409 A1, which is hereby incorporated by reference in its entirety.

In some preferred embodiments, the transfection reagent used in the method of the invention is FectoVIR^{®}. In some embodiments, the transfection reagent comprises (i) at least one compound of general formula (I) or a tautomer, mesomer, racemate, enantiomer, diastereomer, or mixture thereof, or an acceptable salt thereof, and (ii) an acceptable excipient, buffering agent, cell culture medium, or transfection medium: 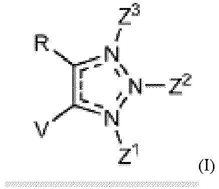 wherein:
Z¹ represents H, X₁-R₃-X₂-P⁺, X₁-R₃-P⁺, X₁-X₂-P⁺, R₃-X₂-P⁺, X₁-P⁺, R₃-P⁺, or X₂-P⁺ ; or Z¹ is absent;
Z₂ represents H, a linear or branched, saturated or unsaturated C₁- C₁₈ alkyl, C₆-C₁₈ aryl, a linear or branched, saturated or unsaturated C₆-C₁₈ aryl-C₁-C₁₈ alkyl, a linear or branched, saturated or unsaturated C₂-C₁₈ heteroalkyl, C₅-C₁₀ heteroaryl, halogen, OH, a linear or branched, saturated or unsaturated C₁-C₁₈ alkylamine, a C₁-C₁₂ alkoxy, a linear or branched, saturated or unsaturated C₁- C₁₈ alkyl-C₁-C₁₂ alkoxy, X₁-R₃-X₂-P⁺, X₁-R₃-P⁺, X₁-X₂-P⁺, R₃-X₂-P⁺, X₁-P⁺, R₃-P⁺, or X₂-P⁺; or Z₂ is absent;
Z₃ represents H, a linear or branched, saturated or unsaturated C₁-C₁₈ alkyl, C₆-C₁₈ aryl, a linear or branched, saturated or unsaturated C₆-C₁₈ aryl-C₁-C₁₈ alkyl, C₅-C₁₀ heteroaryl, a linear or branched, saturated or unsaturated C₂-C₁₈ heteroalkyl, C₂-C₁₈ alkylidene, OH, guanidine, halogen, X₁-R₃-X₂-P⁺, X₁-R₃-P⁺, X₁-X₂-P⁺, R₃-X₂-P⁺, X₁-P⁺, R₃-P⁺, or X₂-P⁺; or Z 3 is absent;
X₁ and X₂, which may be identical or different, represent CO or CH₂ ;
R₃ represents (CH₂)ₘ, (CH₂)ₘ-CHCH₃-(CH₂)ₙ-, (CH₂)ₘ-C(CH₃)₂-(CH₂)ₙ-, (CH₂)ₘ-O-(CH₂)ₙ-, (CH₂)ₘ-S-(CH₂)ₙ-, (CH₂)ₘ-CH₂-O-, with m representing an integer between 1 and 3 and n representing an integer between 1 and 3;
P⁺ represents a graft cationic polymer, which is a polyamine comprising secondary amines, tertiary amines, a mixture of primary and secondary amines, a mixture of primary and tertiary amines, a mixture of secondary and tertiary amines, or a mixture of primary, secondary and tertiary amines;
R or V represents H, a linear or branched, saturated or unsaturated C₁-C₁₈ alkyl or cycloalkyl, a C₆-C₁₈ aryl, a linear or branched, saturated or unsaturated C₆-C₁₈ aryl-C₁-C₁₈ alkyl, a linear or branched, saturated or unsaturated C₂-C₁₈ heteroalkyl, a linear or branched, saturated or unsaturated C₁-C₂₄ ester, a C₅-C₁₀ heterocyclyl, a C₅-C₁₀ heteroaryl, a linear or branched, saturated or unsaturated C₁-C₁₈ alkyl-C₅-C₁₀ heteroaryl, X₁-R₃-X₂-P⁺, X₁-R₃-P⁺, X₁-X₂-P⁺, R₃-X₂-P⁺, X₁-P⁺, R₃-P⁺, or X₂-P⁺;
with the provisos that:
   - at least one of Z₁, Z₂ or Z₃ is present; and
   - only one of Z₁, Z₂, Z₃, R or V represents X₁-R₃-X₂-P⁺, X₁-R₃-P⁺, X₁-X₂-P⁺, R₃-X₂-P⁺, X₁-P⁺, R₃-P⁺, or X₂-P⁺.

The compound of general formula (I) may be prepared according to various methods well known in the art, for example as disclosed in the patent applications WO2021/023796 and WO2021/023798.

In some embodiments, the transfection reagent used in the method of the invention is PEIpro^{®}. In some aspects, the invention provides a rAAV preparation obtainable by the method disclosed herein.

In another aspect of the invention, a use of the rep plasmid, rep-cap plasmid and/or the plasmid system disclosed herein for increasing the potency of rAAV vectors is provided.

The embodiments disclosed herein for the rep plasmid and the plasmid system of the invention can be used for the method of producing recombinant adeno-associated viral vectors as disclosed herein. Features described herein in more detail in connection with the "rep plasmid" and the "plasmid system" embodiments equally apply to the corresponding "method" embodiments.

### SEQUENCE LISTING

This application contains a Sequence Listing which has been submitted electronically and is hereby incorporated by reference in its entirety. Said Sequence Listing file is named 240275EP_Sequence Listing.xml and 38.923 Bytes in size.
SEQ ID NOs: 1, 2, 3, and 4 are exemplary amino acid sequences of the rep78, rep68, rep52, and rep40 proteins, respectively.
SEQ ID NOs: 5, 6, and 7 are exemplary amino acid sequences of the VP1, VP2, and VP3 proteins, respectively.
SEQ ID NOs: 8, 9, and 10 are exemplary amino acid sequences of the AAP, MAAP, and X proteins, respectively.
SEQ ID NO: 11 is an exemplary polynucleotide sequence of the wildtype 3'ITR.
SEQ ID NO: 12 is an exemplary polynucleotide sequence of the RBE derived from a p5 promoter.
SEQ ID NOs: 13, 14, 15, 16, and 17 are exemplary polynucleotide sequences of the MMTV, CGA, DNAH17, AMH, and p5 promoters, respectively.
SEQ ID NOs: 18, 19, 20, and 21, are exemplary polynucleotide sequences of the p19, EF1A, TMBIM6, and GAPDH promoters, respectively.
SEQ ID NO: 22 is an exemplary amino acid sequence of an eGFP fluorescent reporter suitable for use as transgene of the disclosure.
SEQ ID NO: 23 is an exemplary polynucleotide sequence of a rabbit β-globin poly(A) signal sequence.
SEQ ID NO: 24 is an exemplary polynucleotide sequence of the p40 promoter.
SEQ ID NO: 25 is an exemplary AAV2 complete genome.
SEQ ID NO: 26 is an exemplary polynucleotide sequence of an RBE derived from an inverted terminal repeats sequence.

### EXAMPLES

### Example 1:

HEK293 cells in suspension were seeded at 2×10⁶ VCD/mL into SF medium and 24 hours later HEK293 cells were transfected with rep plasmids at 1 µg / 2×10⁶ cells and PEIPro^{®} transfection reagent. Every 24 hours post transfection cells were lysed in Macherey-Nagel RA1 (1% β-mercaptoethanol) Lysis Buffer for RNA analysis. Clarified samples were nuclease digested, RNA purified and reverse transcribed. cDNA was analyzed via ddPCR with primer/probe pairs targeting rep68/rep78 only, all 4 rep mRNAs and GAPDH as internal control. The following rep plasmids were investigated:
- Rep (p5) comprising:
   ∘ coding sequences for the long rep proteins rep78/68 (SEQ ID NO: 1 and 2) driven by their native p5 promoter (SEQ ID NO: 17); and
   ∘ coding sequences for the short rep proteins rep52/40 (SEQ ID NO: 3 and 4) driven by their native p19 promoter (SEQ ID NO: 18);
- Rep (pMMTV) comprising:
   ∘ coding sequences for the long rep proteins rep78/68 (SEQ ID NO: 1 and 2) operatively linked to the heterologous MMTV promoter (SEQ ID NO: 13);
   ∘ coding sequences for the short rep proteins rep52/40 (SEQ ID NO: 3 and 4) driven by their native p19 promoter (SEQ ID NO: 18);
   ∘ a p5-derived RBE (SEQ ID NO: 12); and
   ∘ a heterologous strong rabbit β-globin poly(A) signal (SEQ ID NO: 23).

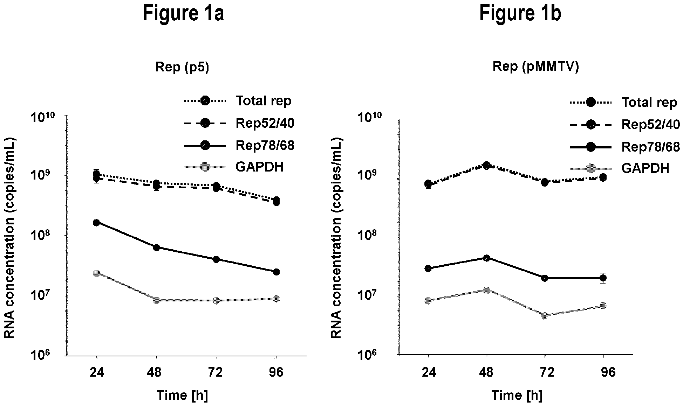

Figures 1a and 1b show that the rep (MMTV) plasmid provides lower long rep protein mRNA concentrations compared to the conventional rep (p5) plasmid comprising the native p5 promoter operatively linked to the long rep protein coding sequence.

Furthermore, the long rep protein mRNA concentrations obtained by the rep (MMTV) plasmid remain stable for a period of 24 to 96 hours. The data therefore demonstrate that the concentration of long rep protein mRNA can be tailored by the heterologous promoter operatively linked to the long rep protein coding sequence.

### Example 2:

HEK293 cells in suspension were seeded at 2×10⁶ VCD/mL into SF medium and 24 hours later HEK293 cells were transfected with rep-cap plasmids, helper plasmids (Aldevron^{©} pHelper), and EGFP transgene plasmids (Plasmidfactory^{©} ssAAV GFP) at 4 ug/10⁶ VCD with PEIPro^{®} transfection reagent. After 72 hours, cells were lysed and potency (TU/L) and viral genome titers (vg/L) were determined. Potency was determined based on eGFP transgene expression in the target cell using the Incucyte^{®} Live-Cell Analysis System and TU/L crude lysate assessment by Poisson distribution. Non-transduced cells were used as negative control. Viral genome titers were determined by ddPCR. The following rep-cap plasmids were investigated:
- Rep-Cap (p5) comprising:
   ∘ coding sequences for the long rep proteins rep78/68 (SEQ ID NO: 1 and 2) driven by their native p5 promoter (SEQ ID NO: 17);
   ∘ coding sequences for the short rep proteins rep52/40 (SEQ ID NO: 3 and 4) driven by their native p19 promoter (SEQ ID NO: 18);
   ∘ coding sequences for the cap proteins V1, VP2, and VP3 (SEQ ID NO: 5, 6 and 7) driven by their native p40 promoter (SEQ ID NO: 24);
   ∘ coding sequences for the accessory proteins AAP, MAAP, and X (SEQ ID NO: 8, 9, and 10) driven by their native p40 promoter (SEQ ID NO: 24).
- Rep-Cap (pMMTV + RBE + polyA) comprising:
   ∘ coding sequences for the long rep proteins rep78/68 (SEQ ID NO: 1 and 2) operatively linked to the heterologous MMTV promoter (SEQ ID NO: 13);
   ∘ coding sequences for the short rep proteins rep52/40 (SEQ ID NO: 3 and 4) driven by their native p19 promoter (SEQ ID NO: 18);
   ∘ coding sequences for the cap proteins V1, VP2, and VP3 (SEQ ID NO: 5, 6 and 7) driven by their native p40 promoter (SEQ ID NO: 24);
   ∘ coding sequences for the accessory proteins AAP, MAAP, and X (SEQ ID NO: 8, 8, and 10) driven by their native p40 promoter (SEQ ID NO: 24);
   ∘ a p5-derived RBE (SEQ ID NO: 12); and
   ∘ a heterologous strong rabbit β-globin poly(A) signal (SEQ ID NO: 23).

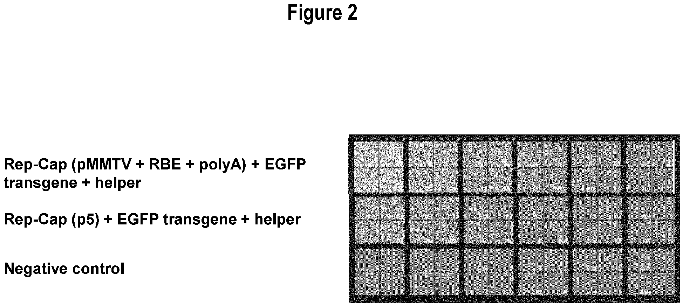

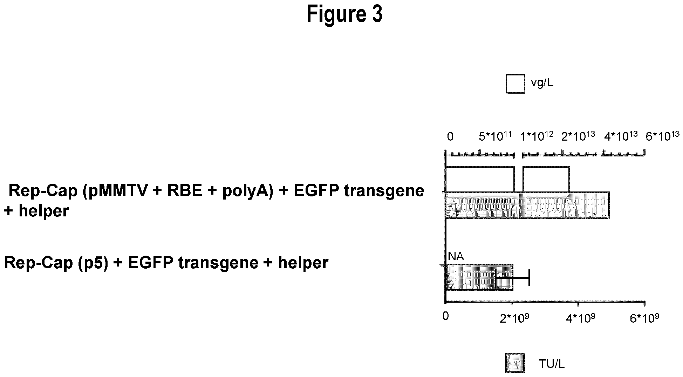

Figures 2 and 3 show that, surprisingly, the potency of rAAVs produced by the rep-plasmid (Rep-Cap (pMMTV + RBE + polyA)) is improved compared to rAAVs produced by a conventional rep plasmid (Rep-Cap (p5)) with long rep protein expression controlled by the native p5 promoter. This demonstrates that tailoring the ratio of long rep proteins and short rep proteins towards low concentrations of long rep proteins and high concentrations of short rep proteins improves rAAV potency.

### Example 3:

HEK293 cells in suspension were seeded at 2×10⁶ VCD/mL into SF medium and 24 hours later HEK293 cells were transfected with rep-cap plasmids, helper plasmids (Sartorius H), and EGFP transgene plasmids (Sartorius ssGFP) at 4 ug/10⁶ VCD with PEIPro^{®} transfection reagent. After 72 hours, cells were lysed and potency (TU/L), capsid titers (cp/L), and viral genome titers (vg/L) were determined. Potency was determined based on eGFP transgene expression in the target cell using the Incucyte^{®} Live-Cell Analysis System and TU/L crude lysate assessment by Poisson distribution. Viral genome titers were determined by ddPCR and capsid titers were determined by Enzyme-linked Immunosorbent Assay (Progen AAV2 Xpress ELISA). The following rep-cap plasmids were investigated:
- Rep-Cap (p5) comprising:
   ∘ coding sequences for the long rep proteins rep78/68 (SEQ ID NO: 1 and 2) driven by their native p5 promoter (SEQ ID NO: 17);
   ∘ coding sequences for the short rep proteins rep52/40 (SEQ ID NO: 3 and 4) driven by their native p19 promoter (SEQ ID NO: 18);
   ∘ coding sequences for the cap proteins V1, VP2, and VP3 (SEQ ID NO: 5, 6 and 7) driven by their native p40 promoter (SEQ ID NO: 24);
   ∘ coding sequences for the accessory proteins AAP, MAAP, and X (SEQ ID NO: 8, 9, and 10) driven by their native p40 promoter (SEQ ID NO: 24).
- Rep-Cap (pMMTV + RBE + polyA) comprising:
   ∘ coding sequences for the long rep proteins rep78/68 (SEQ ID NO: 1 and 2) operatively linked to the heterologous MMTV promoter (SEQ ID NO: 13);
   ∘ coding sequences for the short rep proteins rep52/40 (SEQ ID NO: 3 and 4) driven by their native p19 promoter (SEQ ID NO: 18);
   ∘ coding sequences for the cap proteins V1, VP2, and VP3 (SEQ ID NO: 5, 6 and 7) driven by their native p40 promoter (SEQ ID NO: 24);
   ∘ coding sequences for the accessory proteins AAP, MAAP, and X (SEQ ID NO: 8, 9, and 10) driven by their native p40 promoter (SEQ ID NO: 24);
   ∘ a p5-derived RBE (SEQ ID NO: 12); and
   ∘ a heterologous strong rabbit β-globin poly(A) signal (SEQ ID NO: 23).

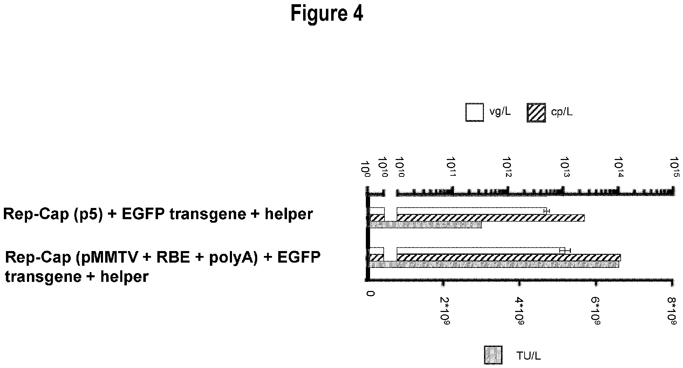

Figure 4 shows, that surprisingly, viral genome titers, capsid titers, and potency are improved for rAAVs obtained by the rep plasmid disclosed herein (Rep-Cap (pMMTV + RBE + polyA)) compared to rAAVs produced by a conventional rep plasmid (Rep-Cap (p5)) with long rep protein expression controlled by the native p5 promoter. In particular, the potency is significantly improved, i.e., by a factor of > 2. This demonstrates that tailoring the ratio of long rep proteins and short rep proteins towards low concentrations of long rep proteins and high concentrations of short rep proteins improves both yield and potency of rAAVs.

### Example 4:

HEK293 cells in suspension were seeded at 2×10⁶ VCD/mL into Ambr15^{®} cell culture biorecators and 24 hours later transfected with rep-cap plasmids, helper plasmids (Aldevron^{©} pHelper), and EGFP transgene plasmids (Plasmidfactory^{©} ssAAV GFP) at 4 ug/10⁶ VCD with FectoVIR^{®} transfection reagent. After 72 hours, cells were lysed and potency (TU/L), viral genome titers (vg/L), and percentage of full capsids (% full) were determined. Potency was determined based on eGFP transgene expression in the target cell using the Incucyte^{®} Live-Cell Analysis System and TU/L crude lysate assessment by Poisson distribution. Viral genome titers were determined by ddPCR and viral capsid titers (cp/L) were determined by Enzyme-linked Immunosorbent Assay (Progen AAV2 Xpress ELISA). The percentage of full capsids was calculated as the ratio of vg/L to cp/L. The following rep-cap plasmids were investigated:
- Rep-Cap (p5) comprising:
   ∘ coding sequences for the long rep proteins rep78/68 (SEQ ID NO: 1 and 2) driven by their native p5 promoter (SEQ ID NO: 17);
   ∘ coding sequences for the short rep proteins rep52/40 (SEQ ID NO: 3 and 4) driven by their native p19 promoter (SEQ ID NO: 18);
   ∘ coding sequences for the cap proteins V1, VP2, and VP3 (SEQ ID NO: 5, 6 and 7) driven by their native p40 promoter (SEQ ID NO: 24);
   ∘ coding sequences for the accessory proteins AAP, MAAP, and X (SEQ ID NO: 8, 9, and 10) driven by their native p40 promoter (SEQ ID NO: 24).
- Rep-Cap (pMMTV + RBE + polyA) comprising:
   ∘ coding sequences for the long rep proteins rep78/68 (SEQ ID NO: 1 and 2) operatively linked to the heterologous MMTV promoter (SEQ ID NO: 13);
   ∘ coding sequences for the short rep proteins rep52/40 (SEQ ID NO: 3 and 4) driven by their native p19 promoter (SEQ ID NO: 18);
   ∘ coding sequences for the cap proteins V1, VP2, and VP3 (SEQ ID NO: 5, 6 and 7) driven by their native p40 promoter (SEQ ID NO: 24);
   ∘ coding sequences for the accessory proteins AAP, MAAP, and X (SEQ ID NO: 8, 9, and 10) driven by their native p40 promoter (SEQ ID NO: 24);
   ∘ a p5-derived RBE (SEQ ID NO: 12); and
   ∘ a heterologous strong rabbit β-globin poly(A) signal (SEQ ID NO: 23).

Figures 5 and 6 show that, surprisingly, the viral genome titers and percentage of full rAAV capsids obtained by the rep plasmid disclosed herein (Rep-Cap (pMMTV + RBE + polyA)) are improved compared to rAAVs produced by a conventional rep plasmid (Rep-Cap (p5)) with long rep protein expression controlled by the native p5 promoter. Furthermore, Figures 7a and 7b show that rAAVs obtained by the rep plasmid disclosed herein (Rep-Cap (pMMTV + RBE + polyA)) are able to transduce a target cell and express a transgene in the target cell. This demonstrates that tailoring the ratio of long rep proteins and short rep proteins towards low concentrations of long rep proteins and high concentrations of short rep proteins improves the yield of functional rAAVs, i.e., rAAVs with full capsids which are able to transduce a target cell and express a transgene in the target cell.

### BRIEF DESCRIPTION OF THE DRAWINGS

Figure 1 depicts the measured mRNA concentrations (copies/mL) of long rep proteins (rep78 and rep68), short rep proteins (rep52 and rep40), combined concentrations of long rep and short rep proteins (total rep), and GAPDH (internal standard) over a period of 24 to 96 hours. Figure 1a depicts the mRNA concentrations obtained by a conventional rep plasmid (Rep (p5)) comprising the native p5 promoter operatively linked to the long rep protein coding sequence. Figure 1b shows the mRNA concentrations obtained by the rep plasmid disclosed herein (Rep(pMMTV)) comprising a heterologous promoter with decreased promoter strength (compared to p5) operatively linked to the long rep protein coding sequence.
Figure 2 depicts the potency of a conventional rep-cap plasmid (Rep-Cap comprising the native p5 promoter operatively linked to the long rep protein coding sequence (Rep-Cap (p5)) and of a rep plasmid disclosed herein (Rep-Cap comprising a heterologous promoter with reduced promoter strength (pMMTV) operatively linked to the long rep protein coding sequence, an RBE, and a strong polyA signal (Rep-Cap (pMMTV + RBE + polyA)). Potency was measured in a three-plasmid system comprising the rep-cap (p5) or rep-cap (pMMTV + RBE + polyA) plasmids, a transgene plasmid comprising enhanced green fluorescent protein (eGFP) as the transgene (EGFP transgene), and a helper plasmid (helper). Potency was analyzed by fluorescence microscopy based on eGFP transgene expression in the target cell. Non-transduced cells were used as negative control.
Figure 3 depicts the measured concentrations of viral genomes (vg/L) and the measured potency (TU/L) of a conventional rep-cap plasmid (Rep-Cap comprising the native p5 promoter operatively linked to the long rep protein coding sequence (Rep-Cap (p5)) and of a rep plasmid as disclosed herein (Rep-Cap comprising a heterologous promoter with reduced promoter strength (pMMTV) operatively linked to the long rep protein coding sequence, an RBE, and a strong polyA signal (Rep-Cap (pMMTV + RBE + polyA)).Concentrations of viral genomes and transducing titers were measured in a three-plasmid system comprising the rep-cap (p5) or rep-cap (pMMTV + RBE + polyA) plasmids, a transgene plasmid comprising eGFP as the transgene (EGFP transgene), and a helper plasmid (helper).
Figure 4 depicts measured concentrations of viral genomes (vg/L) and viral capsid proteins (cp/L) and the measured potency (TU/L) of a conventional rep-cap plasmid (Rep-Cap comprising the native p5 promoter operatively linked to the long rep protein coding sequence (Rep-Cap (p5)) and of a rep plasmid disclosed herein (Rep-Cap comprising a heterologous promoter with reduced promoter strength (pMMTV) operatively linked to the long rep protein coding sequence, an RBE, and a strong polyA signal (Rep-Cap (pMMTV + RBE + polyA)). Concentrations of viral genomes and viral capsid proteins and transducing titers were measured in a three-plasmid system comprising the rep-cap (p5) or rep-cap (weakP + RBE + polyA) plasmids, a transgene plasmid comprising eGFP as the transgene (EGFP transgene), and a helper plasmid (helper).

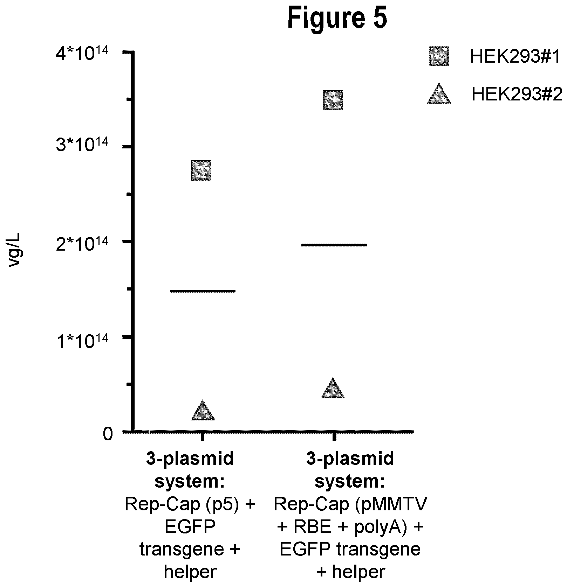

Figure 5 depicts the measured concentrations of viral genomes (vg/L) obtained by a three-plasmid system. The three-plasmid system comprises either a conventional rep-cap plasmid (Rep-Cap comprising the native p5 promoter operatively linked to the long rep protein coding sequence (Rep-Cap (p5)) or a rep plasmid disclosed herein (Rep-Cap comprising a heterologous promoter with reduced promoter strength (pMMTV) operatively linked to the long rep protein coding sequence, an RBE, and a strong polyA signal (Rep-Cap (pMMTV + RBE + polyA)), and further comprises a transgene plasmid comprising eGFP as the transgene (EGFP transgene) and a helper plasmid (helper). The concentrations of viral genomes were measured for two HEK293 clones (HEK293#1, HEK293#2).

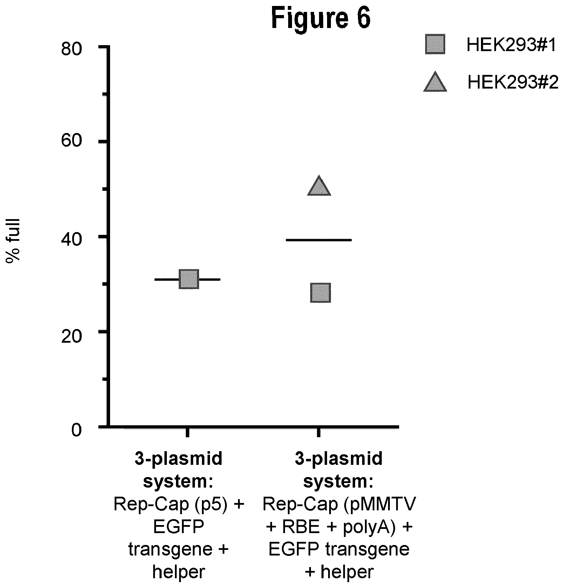

Figure 6 depicts the percentage of full rAAV capsids (% full) obtained by a three-plasmid system. The three-plasmid system comprises either a conventional rep-cap plasmid (Rep-Cap comprising the native p5 promoter operatively linked to the long rep protein coding sequence (Rep-Cap (p5)) or a rep plasmid disclosed herein (Rep-Cap comprising a heterologous promoter with reduced promoter strength (pMMTV) operatively linked to the long rep protein coding sequence, an RBE, and a strong polyA signal (Rep-Cap (pMMTV + RBE + polyA)), and further comprises a transgene plasmid comprising eGFP as the transgene (EGFP transgene) and a helper plasmid (helper). The percentage of full rAAV capsids was measured for two HEK293 clones (HEK293#1, HEK293#2).

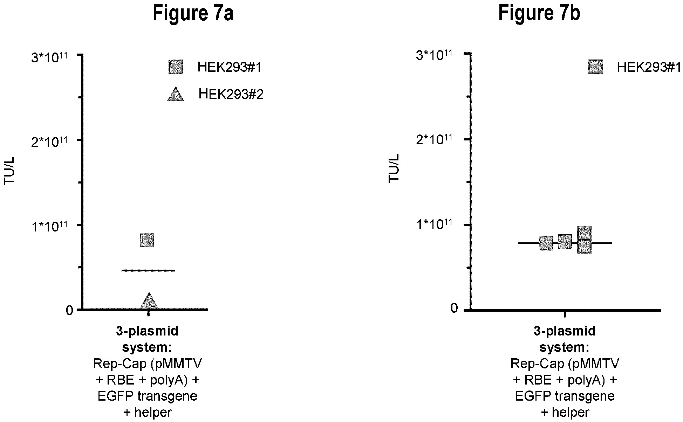

Figures 7a and 7b depict the measured potency (TU/L) obtained by a three-plasmid system comprising a rep plasmid disclosed herein (Rep-Cap comprising a heterologous promoter with reduced promoter strength (pMMTV) operatively linked to the long rep protein coding sequence, an RBE, and a strong polyA signal (Rep-Cap (pMMTV + RBE + polyA)), a transgene plasmid comprising eGFP as the transgene (EGFP transgene), and a helper plasmid (helper). The transducing titers were measured for two HEK293 clones (HEK293#1, HEK293#2) or one HEK293 clone (HEK293#1).

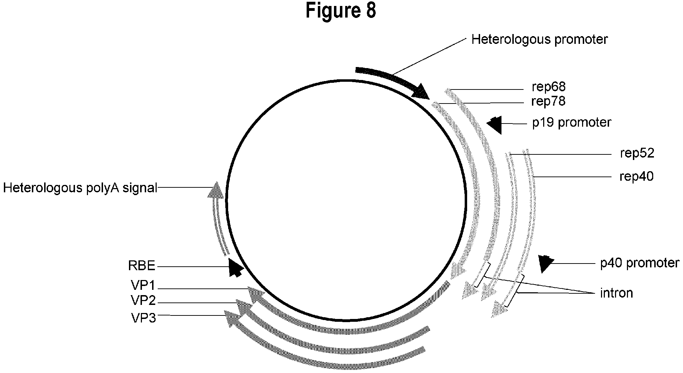

Figure 8 depicts a schematic representation of an exemplary embodiment of the rep plasmid disclosed herein. The rep plasmid depicted in Figure 8, comprises a heterologous promoter, long rep protein (rep68 and rep78) and short rep protein (rep40 and rep52) coding sequences, cap protein coding sequences (VP1-VP3), an RBE, and a heterologous polyadenylation signal sequence (heterologous polyA signal), and p19 and p40 promoters. The p40 promoter regulates the transcription of the accessory protein coding sequences.

## Claims

1. A rep plasmid comprising:
(i) at least one long adeno-associated virus replication protein coding sequence encoding at least one long functional rep protein;
(ii) at least one short adeno-associated virus replication protein coding sequence encoding at least one short functional rep protein; and
(iii) at least one heterologous element, wherein following transcription the at least one heterologous element results in a ratio of long adeno-associated virus rep mRNA to short adeno-associated virus rep mRNA of about 1:5 to about 1:5000.

2. The rep plasmid according to claim 1, wherein after transcription of the rep protein coding sequence, the ratio of long adeno-associated virus rep protein mRNA to short adeno-associated virus rep protein mRNA is
(i) about 1:5 to about 1:300, preferably about 1:5 to 1:250, more preferably about 1:5 to about 1:200, even more preferably about 1:5 to about 1:150, and most preferably about 1:5 to about 1:100, optionally, wherein the long adeno-associated virus rep protein mRNA level is high; and/or
(ii) about 1:5 to about 1:5000, preferably about 1:20 to about 1:2500, more preferably about 1:50 to about 1:2000, and most preferably about 1:100 to about 1:1500, optionally, wherein the long adeno-associated virus rep protein mRNA level is low.

3. The rep plasmid according to any one of claims 1 or 2, wherein the at least one heterologous element is a heterologous promoter, preferably operatively linked to the long adeno-associated virus replication protein coding sequence.

4. The rep plasmid according to any one of claims 1-3, wherein the rep plasmid comprises one or more polyadenylation signal sequences.

5. The rep plasmid according to any one of claims 1-4, wherein the at least one heterologous element is a heterologous polyadenylation signal sequence.

6. The rep plasmid according to any one of claims 1-5, wherein the rep plasmid comprises at least one adeno-associated virus capsid protein coding sequence encoding at least one functional cap protein.

7. A plasmid system for producing an adeno-associated virus particle comprising:
(i) the rep plasmid of any one of claims 1-6; and
(ii) a transgene plasmid.

8. The plasmid system according to claim 7, wherein the plasmid system further comprises a helper plasmid.

9. The plasmid system according to any one of claims 7 or 8, wherein the plasmid system further comprises a cap plasmid, preferably comprising at least one adeno-associated virus capsid protein coding sequence encoding at least one functional cap protein.

10. The plasmid system according to any one of claims 7-9, wherein the rep plasmid comprises at least one adeno-associated virus capsid protein coding sequence encoding at least one functional cap protein.

11. A rep-cap plasmid comprising:
(i) at least one long adeno-associated virus replication protein coding sequence encoding at least one long functional rep protein;
(ii) at least one short adeno-associated virus replication protein coding sequence encoding at least one short functional rep protein;
(iii) at least one adeno-associated virus capsid protein coding sequence encoding at least one functional cap protein; and
(iv) at least one heterologous element, wherein following transcription the at least one heterologous element results in a ratio of long adeno-associated virus rep mRNA to short adeno-associated virus rep mRNA of about 1:5 to about 1:5000.

12. A stable or transient cell expression system comprising the plasmid system of any one of claims 7-10 and a cell line.

13. A cell comprising the rep plasmid of any one of claims 1-6, the plasmid system of any one of claims 7-10, or the rep-cap plasmid of claim 11.

14. A kit comprising the stable or transient cell expression system of claim 12, or a cell of claim 13, and a cell culture medium.

15. A method of producing recombinant adeno-associated viral vectors comprising:
(i) Transfecting cells with the rep plasmid of any one of claims 1-6, the plasmid system of any one of claims 7-10, or the rep-cap plasmid of any one of claims 11;
(ii) culturing the transfected cells to produce said adeno-associated viral vectors; and
(iii) isolating said recombinant adeno-associated viral vectors.
